(19) Europäisches Patentamt
European Patent Office
Office européen des brevets

(11) **EP 4 452 260 B1**

(12) # EUROPEAN PATENT SPECIFICATION

(45) Date of publication and mention of the grant of the patent:
**28.01.2026 Bulletin 2026/05**

(21) Application number: **22843707.5**

(22) Date of filing: **21.12.2022**

(51) International Patent Classification (IPC):
***A61K 31/401*** *(2006.01)* ***A61P 27/02*** *(2006.01)*
***A61K 38/05*** *(2006.01)*

(52) Cooperative Patent Classification (CPC):
**A61K 31/401; A61K 38/05; A61P 27/02**

(86) International application number:
**PCT/EP2022/087342**

(87) International publication number:
**WO 2023/118366 (29.06.2023 Gazette 2023/26)**

(54) **GAP JUNCTION MODULATORS AND THEIR USE FOR THE TREATMENT OF AGE-RELATED MACULAR DEGENERATION**

GAP-JUNCTION-MODULATOREN UND DEREN VERWENDUNG ZUR BEHANDLUNG VON ALTERSBEDINGTER MAKULADEGENERATION

MODULATEURS DE JONCTIONS COMMUNICANTES ET LEUR UTILISATION POUR LE TRAITEMENT DE LA DÉGÉNÉRESCENCE MACULAIRE LIÉE À L'ÂGE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **22.12.2021 US 202163292783 P**

(43) Date of publication of application:
**30.10.2024 Bulletin 2024/44**

(73) Proprietor: **Breye Therapeutics ApS**
**2970 Hørsholm (DK)**

(72) Inventors:
- **MOURITZEN, Ulrik**
  **2960 Rungsted Kyst (DK)**
- **YOUNG, Bradford**
  **Annapolis, MD 21403 (US)**

(74) Representative: **Mewburn Ellis LLP**
**Aurora Building**
**Counterslip**
**Bristol BS1 6BX (GB)**

(56) References cited:
**EP-B1- 3 618 847**

- **MAT NOR MOHD NASIR ET AL: "Connexin Hemichannel Block Using Orally Delivered Tonabersat Improves Outcomes in Animal Models of Retinal Disease", NEUROTHERAPEUTICS, SPRINGER INTERNATIONAL PUBLISHING, CHAM, vol. 17, no. 1, 21 October 2019 (2019-10-21), pages 371 - 387, XP037014143, ISSN: 1933-7213, [retrieved on 20191021], DOI: 10.1007/S13311-019-00786-5**
- **GUO CINDY X. ET AL: "Connexin43 Mimetic Peptide Improves Retinal Function and Reduces Inflammation in a Light-Damaged Albino Rat Model", INVESTIGATIVE OPTHALMOLOGY & VISUAL SCIENCE, vol. 57, no. 10, 3 August 2016 (2016-08-03), US, pages 3961 - 3973, XP093030876, ISSN: 1552-5783, DOI: 10.1167/iovs.15-16643**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the Invention

The present invention relates to gap junction-dependent cellular modulators and their use of the treatment or prevention of age-related macular degeneration (AMD). The present invention further relates to pharmaceutical compositions adapted for the delivery of gap junction-dependent cellular modulators to the eye.

### Background of the Invention

Age-related macular degeneration (AMD) is one of the most important causes of vision loss in elderly people. AMD is a progressive and degenerative disorder of the central retina that results in loss of visual acuity and even blindness in patients with damaged maculas. Given that age is a major risk factor for AMD, the prevalence and severity of this disease are likely to increase as human life expectancy increases, such as in developed countries (Bandello *et al.,* 2017).

AMD is a disorder of the central retina with a multifactorial etiopathology and can be classified as dry AMD (d-AMD; also known as non-neovascular AMD) or neovascular AMD (n-AMD; also known as wet AMD), depending on the presence of choroidal neovascularization (CNV) - a hallmark of n-AMD and a distinguishing feature from non-neovascular dry AMD. Clinically, AMD generally presents in two forms: a non-exudative "dry" form (i.e. d-AMD) and an exudative, neovascular "wet" (i.e. wet AMD) form. Over 80% of AMD patients present with d-AMD, which is also termed atrophic AMD, and is characterized by thinning of the macula, drusen deposits, and early leakage in the retina. The advanced form of dry AMD, termed geographic atrophy (GA), presents with extensive loss of the retinal pigment epithelium (RPE) and photoreceptors, and this severe stage of disease can lead to irreversible blindness in up to 20% of patients. Currently, there are no effective treatments for d-AMD, and as it is very difficult to restore the RPE or photoreceptors once they have sustained substantial damage or died-off, especially in the advanced stages of disease (Bandello *et al.,* 2017).

A sub-population (10-20%) of d-AMD patients develop wet AMD, generally later in the course of disease due to abnormal blood vessels grow in the retina. The leading eye injection treatments for wet AMD are the same market therapies (e.g. Eylea® and Lucentis®) as those used to treat diabetic retinopathy (DR) patients with diabetic macular edema (DME). The aberrant neovascularization that can characterize both proliferative DR and wet AMD is associated with dramatic increases in vascular leakage and edema that damages the retina and macula leading to debilitating vision loss. As in patients with proliferative DR and DME, the leading anti-VEGF injection treatments, Eylea® and Lucentis® have been shown to effectively reduce this pathological angiogenesis and improve visual acuity with optimal treatment responses observed in only approximately 40% of the patients. In addition, these treatments require years of burdensome injections with limited durability of response. As such, there is a large unmet need for more effective treatments of patients with both wet and dry AMD that can effectively treat these diseases at earlier and less severe disease stages before disease progression and permanent and debilitating vision loss occurs.

Clinical conditions without overt functional or vision loss, such as early and/or intermediate stages of AMD, are characterized by deposition of drusen and/or retinal pigment epithelium (RPE) dysfunction or alterations in the macular area. In the late stages, the disease may progress to either geographic atrophy (GA) or neovascular AMD (n-AMD). Geographic atrophy (GA) secondary to d-AMD occurs when the RPE begins to degenerate in the region of the macula leading to rod and cone photoreceptor (PR) cell death and eventual central vision loss and blindness due to the substantial loss of photoreceptors in the eye (Naylor *et al.,* 2020).

#### Pathogenesis

The exact pathophysiological mechanisms behind AMD have yet to be determined. However, it is clear AMD is a multifactorial pathology, in which genetic and environmental risk factors play a crucial role (Bandello *et al.,* 2017).

Several pathways have been identified that seem to play an important role in the pathogenesis of d-AMD, including oxidative stress, deposits of lipofuscin, chronic inflammation, and choroidal blood flow insufficiency. These pathways represent possible targets for new therapies (Bandello *et al.,* 2017).

For example, the blood retina barriers (BRB) are fundamental in establishing and maintaining a suitable environment for optimum retinal function (Naylor *et al.,* 2020). The BRB comprises of the inner blood retina barrier (iBRB) and the outer blood retina barrier (oBRB). The iBRB is composed of retinal endothelial cells and pericytes that form highly regulated cell-cell couplings via specialized protein complexes between the endothelial cells and the pericytes to maintain barrier function. The oBRB is composed of retinal pigment epithelial (RPE) cells that form highly regulated cell-cell couplings via specialized protein complexes between the RPEs to maintain barrier function. These specialized protein complexes localized to the plasma membranes of retinal endothelial, pericytes and epithelial cells, includes Gap Junctions, Tight Junctions (TJs) and Adherens Junctions.

The RPE is a critical homeostatic component of the oBRB and separates the Bruch's membrane of the choroid

and the photoreceptor layer of the neural retina. The epithelial cells forming the RPE monolayer are pigmented, highly polarized epithelial cells with their apical surface facing the photoreceptors and basal surface attached to the Bruch's membrane. These epithelial cells are compactly arranged with the aid of actin-dependent cell-cell junctions, in particular TJs. The oBRB acts to regulate and filter molecular movement of solutes and nutrients from the choroid to the sub-retinal space and is critical for maintaining retinal homeostasis. The tightly compact monolayer of RPE cells that form the oBRB play an important role in the development and maintenance of the retina including secretion of growth factors, limiting the entry of large and toxic molecules in the retina from the blood stream, regulating the transport of nutrients and fluids into and from the retina, and maintaining the biogeneric health of photoreceptors. A loss of RPE integrity and dysfunction of the oBRB has been associated with several ocular diseases including AMD, DR and DME. Impairment of either of these barriers can lead to the accumulation of fluid, blood-borne proteins, and other potentially toxic solutes within the retina (Naylor *et al.,* 2020).

The RPE can be divided into apical and basolateral sides. The apical surface is in direct contact with the photoreceptor outer segment (POS) and the basolateral side acts as a barrier in its interaction with the highly permeable and highly perfused choriocapillaris of the choroid (Naylor *et al.,* 2020). The RPE exhibits three kinds of intercellular junctions: TJs, Adherens Junctions and Gap Junctions. TJs form a gate or barrier that regulates the paracellular diffusion of solutes and nutrients in the RPE. Adherens Junctions provide strong mechanical attachment between adjacent RPE cells. Gap Junctions allow for cell-to-cell communication and support cell-cell coupling between RPE cells and within the RPE monolayer by associating with TJs (Obert *et* al., 2017).

TJs connecting neighbouring RPE cells block the movement of plasma components and toxic molecules into the retina as well as allowing for a controlled flow of fluid and solutes across an osmotic gradient from the choroidal vasculature to the outer retina. The retina has the highest oxygen consumption per weight of any tissue in the body and the BRB (both outer and inner) is essential in providing a distinct and regulated source of nutrients to facilitate for this high metabolic rate. TJs at both the iBRB and oBRB are complex, dynamic structures and in the context of these barriers, the integrity of these TJs is crucial to sight.

Specifically, the oBRB allows for the survival of the photoreceptors (PRs) by supporting essential functions including filtering and transport of nutrients and photoreceptor outer segment (POS) phagocytosis. Other important functions of the RPE include absorption of out-of-focus and scattered light, retinal adhesion, and vitamin A transport and processing, and re-isomerisation of all-trans-retinal to 11-cis retinal, which is crucial for the visual cycle (Naylor *et al.,* 2020). Therefore, the RPE is essential for visual function - a failure of any one of these functions can lead to degeneration of the retina, loss of visual function, and ultimately blindness.

TJs in concert with Gap Junctions and Adherens Junctions allow for high degrees of selectivity in paracellular barrier function in both the iBRB and the oBRB. TJs are unique assemblies of transmembrane proteins and peripheral cytoplasmic proteins. Transmembrane proteins include the claudins, occludins, the MARVEL (Mal and related proteins for vesicle trafficking and membrane link) family and junctional adhesion molecules (JAMs), which span the plasma membrane. Peripheral cytoplasmic proteins such as zonula occludens-1 (ZO-1), -2 (ZO-2) and -3 (ZO-3) anchor these transmembrane proteins to the cytoskeleton, along with Gap Junctions and Adherens Junctions, and are vital in the initial formation and distinct organization of TJs (Naylor *et al.,* 2020).

The connexin subunit of Gap Junctions, which are critical for intercellular communication and contribute to cell-cell coupling are tetraspan transmembrane proteins. Twenty-one connexin isoforms have been identified in the human genome, each with distinct spatial and temporal expression patterns (Obert *et al.,* 2017). Six connexins assemble into one connexon (hemichannel), while two connexons from adjacent cells dock to form a gap junction channel - the gap junction itself is an aggregate of such channels. Various heteromeric configurations of different connexin proteins can assemble into connexon hemichannels, leading to Gap Junctions with distinctive communication properties. For example, TJs contain at least 40 proteins, some of which are transmembrane and mediate intercellular adhesion; and others are intracellular scaffold proteins that link junctional components to the cytoskeleton. Adherens Junctions have adhesive elements that are cadherin receptors that bridge the gap between neighbouring cell membranes through homophilic interactions, with catenins as the main scaffolding proteins that tether the mechanical junctions to the cytoskeleton (Obert *et al.,* 2017).

ZO-1 is a scaffolding protein common to all three junction types, anchoring the junctional macromolecular complexes to cytoplasmic actin. ZO-1 belongs to the family of membrane-associated guanylate kinase-like proteins (MAGUK) and incorporates three PDZ domains: an SH3 domain, a GUK domain and a proline-rich domain at the C-terminus (Obert *et al.,* 2017). The binding of Connexin 43 (Cx43) to the PDZ2 domain of ZO-1 regulates the size and stability of Gap Junction channel aggregates. ZO-1 governs the cellular distribution of Cx43, providing a control point for dynamic switching between gap junctional communication and non-junctional (hemichannel) communication at the perinexus - a specialized membrane domain at the periphery of gap junction channel aggregates. ZO-1 disruption in a functional epithelial monolayer results in a loss of barrier function and a reorganization of apical actin and myosin (Obert *et al.,* 2017).

Cx43 is the main connexin responsible for the formation of Gap Junctions in retinal endothelial, pericyte and

epithelial cells, which mediate communication between cells by permitting the passage of small molecules for homeostatic processes such as growth, repair and survival. Six connexin monomers form a hemichannel (HC) which undocked under normal conditions is closed, while docking of two HCs from neighbouring cells results in the formation of a Gap Junction which opens during physiologic conditions to allow exchange of cellular contents (Coutinho *et al.,* 2020). During pathology, however, normally closed, undocked HC are stimulated to open to the extracellular environment eventually resulting in cell death. Sudden tissue reperfusion during open Cx43 HC states drastically increases cell death and tissue damage as cells are unable to cope with the rapid ionic influx.

Hypoxia is often associated with the production of pro-inflammatory cytokines as well as the overexpression of proteins such as the vascular endothelial growth factor (VEGF), Connexin 43 (Cx43) and Syndecan-4 (Coutinho *et al.,* 2020). The RPE expresses Cx43 as the most abundantly expressed connexin (Obert *et al.,* 2017). Furthermore, VEGF is known to disrupt ZO-1 organization, resulting in tight junction disassembly and increased monolayer permeability. Additionally, *in vitro* studies using endothelial cells show that VEGF-mediated disruption of Gap Junction communication is correlated with changes in Cx43 phosphorylation (Coutinho *et al.,* 2020).

For example, in neovascular AMD (n-AMD), unregulated growth of poorly formed blood vessels, known as choroidal neovascularization (CNV), results in haemorrhage within the retina leading to tissue ischaemia (Coutinho *et al.,* 2020). To compensate for the disruption in blood/oxygen supply, VEGF is overexpressed by the retinal pigment epithelium (RPE), which contributes to the blood-retinal barrier (BRB) between the vascular choroid and the neural retina (Coutinho *et al.,* 2020). This VEGF overexpression perpetuates the formation of leaky blood vessels, which introduces more inflammatory factors to the environment and causes RPE barrier dysfunction and cell death due to hypoxia, ultimately permitting blood vessel growth into the retina and leading to vision loss.

In the aging eye, extracellular material is deposited in Bruch's membrane and as a result, the thickness of Bruch's membrane increases and permeability decreases (Naylor *et al.,* 2020). Aging results in an accumulation of oxidative insults and a concomitant decrease in protective mechanisms. The accumulation of lipofuscin in the RPE has been suggested to act as a starting point (Naylor *et al.,* 2020). Oxidative damage is thought to be the initial trigger for age-related degenerative diseases such as AMD (Naylor *et al.,* 2020). The RPE's adaptive response to stress in patients with AMD becomes dysregulated and an increasing imbalance of protective and toxic factors contributes to macular damage and the development of retinal lesions (Naylor *et al.,* 2020). The RPE's capacity to absorb light energy is reduced, which is thought to be an important factor in the cascade of events leading to AMD (Naylor *et al.,* 2020).

The integrity of the outer blood retina barrier (oBRB) keeps the choroidal vascular from invading the retina and changing d-AMD into wet AMD. The loss of this RPE-RPE attachment can induce VEGF overexpression (Naylor *et al.,* 2020). Age-associated RPE degeneration, tears, drusen formation, or apoptosis can also cause a loss of the RPE-RPE attachment.

<u>Diagnosis and Treatments</u>

For diagnostic tools, there are numerous available examinations in clinics to monitor morphological changes in the retina, RPE and choroid of d-AMD patients. For example, fundus autofluorescence and optical coherence tomography (OCT) are considered the most useful tools in the diagnosis and follow-up of d-AMD alterations, including the monitoring of atrophy area progression. Another example is OCT-angiography, a novel imaging tool that may add further information in patients affected by d-AMD. OCT can also be used to diagnose and monitor macular edema as observed in wet AMD (Bandello *et al.,* 2017).

Recently, clinics for intravitreal injections of anti-vascular endothelial growth factor (anti-VEGF) drugs have been introduced and new therapies have been developed targeting vessel maturation and remodeling have revolutionized the natural history of the disease. These intravitreal agents that block VEGF have revolutionised the care of patients with wet AMD, decreasing growth, and leakage from CNV lesions, and preventing moderate and severe visual loss (Naylor *et al.,* 2020).

However, there is currently no approved therapy for geographic atrophy (GA), the late form of d-AMD, because there is no treatment that can restore the damage of retinal pigment epithelium (RPE) or photoreceptors. For this reason, current treatment approaches in d-AMD are only likely to prevent and/or slow down the progression of existing atrophy (Bandello *et al.,* 2017).

Cx43 hemichannel (HC) blockers have been shown to prevent vessel leak, support repair of leaky blood vessels and promote tissue repair in numerous animal models (Coutinho *et al.,* 2020). In chronic hypoxic or inflammatory conditions, Cx43 HCs have been referred to as "pathologic pores" as they are responsible for the activation of the inflammatory cascade via the nod-like receptor family pyrin domain containing 3 (NLRP3) inflammasome complex leading to the production of inflammatory cytokines, and thus perpetuating the inflammatory environment (Coutinho *et al.,* 2020). Blocking open Cx43 HC during injury using Cx43 mimetic peptides such as Gap27 and Pepide5 has been shown to promote cell survival and tissue repair in cardiac, spinal cord injury and ocular models. However, one concern with these peptides is their action on the extracellular motifs of Cx43, potentially affecting gap junction function required for cell

survival when used at high concentrations and/or long exposure periods (Coutinho *et al.,* 2020).

Gap19 is a HC blocker derived from the second cytoplasmic loop of Cx43, which does not interfere with Gap Junction function. However, it requires entering the cell in order to bind to the corresponding sequence of the cytoplasmic tail of Cx43 (Coutinho *et al.,* 2020). Due to its poor cell penetration, high concentrations have previously been used but with limited efficacy. Cell-penetrating peptides (CPP) have therefore been explored to improve the transport of cargo molecules across the cell membrane. For example, Xentry, a CPP derived from the X-protein of the hepatitis B virus, has been shown to improve transport of a range of molecules into cells via endocytic mechanisms by binding to cell surface - expressed Syndecan-4 (Coutinho *et al.,* 2020). As Syndecan-4 is not expressed on circulating monocytes and erythrocytes, sequestration by the circulation, if delivered systemically, is prevented, while uptake into Syndecan-4 overexpressing cells is increased. Coutinho *et al.* found that conjugation of Xentry to Gap19 (XG19) could increase the cellular uptake of Gap19 to block Cx43 HC-mediated injury in hypoxic cells at low peptide concentrations (Coutinho *et al.,* 2020).

Obert *et al.* (2017) hypothesized that targeting ZO-1 signalling would maintain BRB integrity and reduce RPE pathophysiology by stabilizing Gap Junctions and/or TJs. They developed a connexin 43-based peptide mimetic, Alpha Connexin carboxy-Terminal 1 ($\alpha$CT1), to competitively block interactions at the PDZ2 domain of ZO-1, thereby inhibiting ligands that selectively bind to this domain. $\alpha$CT1 mimics the Cx43 COOH-terminal PDZ binding domain, which is different from several other Cx43 mimetics that are thought to cause a reduction in Cx43 channel activity or that target the Cx43 microtubule-binding domain, reducing hemichannel activity. $\alpha$CT1 was thought to maintain BRB integrity and reduce RPE pathophysiology by stabilizing Gap Junctions and/or TJs. In the experiments, RPE-cell barrier dysfunction was generated in mice using laser-photocoagulation triggering choroidal neovascularization (CNV), or bright-light exposure leading to morphological damage. The $\alpha$CT1 treatment reduced CNV development and fluid leakage as determined by optical coherence tomography, and damage was correlated with disruption in cellular integrity of surrounding RPE cells. Pre-treatment with $\alpha$CT1 via eyedrops prevented light-damage, which usually significantly disrupts RPE cell morphology. *In vitro* experiments using RPE and Madin-Darby Canine Kidney (MDCK) monolayers indicated that $\alpha$CT1 stabilizes Tight Junctions, independent of its effects on Cx43. Taken together, stabilization of intercellular junctions by $\alpha$CT1 was effective in ameliorating RPE dysfunction in models of AMD-like pathology. Obert *et al.* concluded that $\alpha$CT1-mediated stabilization of tight junctions may serve as a new treatment for both wet and d-AMD.

King *et al.* (2021) also provides a review of different Cx43 targeting molecules and mentions danegaptide. However, the other peptides disclosed therein do not effectively block hemichannels and stabilize cell-cell coupling via Gap Junctions under stressed conditions in AMD simultaneously.

Accordingly, there is a need in the art for further treatments for age-related macular degeneration (AMD), and especially dry AMD (d-AMD), particularly before it progresses into advanced geographic atrophy (GA) or wet AMD or neovascular AMD (n-AMD).

## Summary of the Invention

The present invention is defined by the claims. Any subject-matter falling outside the scope of the claims is provided for information only.

Methods of treatment of the human body by therapy, referred to in this description, are not part of the present invention as such, but are described here in relation to compounds and pharmaceutical compositions for use in said methods for treatment of the human body by therapy, according to the present invention.

Broadly, the present invention relates compounds that are Gap Junction-dependent cellular modulators, such as Gap Junction intercellular communication (GJIC) modulators, and cell-cell coupling modulators for use in methods of treatment or prevention of age-related macular degeneration (AMD), typically in human patients. The AMD may be dry AMD (d-AMD), advanced GA, wet AMD, or neovascular AMD (n-AMD). In some preferred aspects, the present invention is particularly concerned with the treatment or prevention of dry AMD (d-AMD), particularly just before the condition worsens to a point where the patient develops wet AMD or advanced GA. Although the role of Gap Junction intercellular communication (GJIC) has been extensively studied in various tissues, the present invention concerns its involvement in structural integrity of cell-cell coupling and dysfunction of the outer BRB, including barrier integrity of the RPE in patients with AMD. Gap Junction channels allow the passage of ions, nutrients, and other signaling molecules (up to 1 kDa) between neighboring cells. Importantly, connexin 43 (Cx43)-mediated GJIC plays a critical role in regulating cell growth, vascular tightness, barrier integrity, and cell death in the retina, and is thus an integral factor in maintaining retinal metabolic and vascular homeostasis. Cx43 is abundantly expressed in the retina, which suggests a substantial amount of Gap Junction dependent coupling, especially in both the iBRB and oBRB structures. However, under oxidative stress, ischemic stress, hypoxia, high glucose and diabetic conditions, Cx43-dependent cell-cell coupling is down regulated and functionality impaired, thereby compromising Gap Junction activity in the RPEs and the retinal vascular cells. Moreover, oxidative stress, ischemic stress, hypoxia, high glucose- and diabetes-induced Cx43 downregulation and decreased GJIC activity plays a critical role in the increased RPE and vascular cell death in the retinas of diabetic mice, rats, and humans exhibiting a significant increase in the number of acellular capillaries and pericyte loss.

Moreover, it is known that blood vessels, including those present in the eye, are composed of two interacting cell types (Song *et al.,* 2005). Thus, endothelial cells form an inner lining of the vessel wall, while pericytes surround the surface of the vascular tube, supporting and maintaining vessel structure and barrier integrity, and help to inhibit leakage from the vessel. Pericytes are therefore functionally significant because when vessels lose pericytes, they become haemorrhagic and hyperdilated, leading to conditions such as vascular leakage and edema as the fluid leakage increases beyond the resorptive capacity of the RPE, which leads to loss of vision and ultimately blindness. In addition to the effect of Gap Junction modulators on retinal endothelial cells and RPEs, the present application shows that barrier integrity of the RPE can be protected from oxidative and high glucose stress, and outer retinal thickness swelling in a rat, diabetic retinopathy model can be significantly improved by administration of a Gap Junction modulator.

Furthermore, the combination of maintaining structural cell-cell coupling and closure of hemichannels provide a unique and improved advantage of the compounds described herein, such as danegaptide, over the previously disclosed therapeutic benefit of molecules that predominantly target closure of hemichannels, such as Peptide5 and Tonabersat. Both compounds have been suggested to be useful in the treatment of age-related macular degeneration by blocking Cx43 hemichannels (Mat et al., 2019; Guo et al.; 2016).

Without wishing to be bound by theory, the inventors have observed in BRB models of AMD and DME that suggest that the MoA (mode-of-action) for the effects of the compounds described herein, such as danegaptide, are primarily due the compound's treatment / protective effects on Gap Junction (in concert with TJs) dependent cell-cell coupling in the presence of high glucose, reactive oxygen species (RoS) and metabolic / bioenergenic (i.e. oxidative phosphorylation (OxPos), tricarboxylic acid cycle (TCA) and glycolysis) and other cell stressors. The compounds described herein may also prevent pathological opening of Gap Junction hemichannels in AMD that can cause cell loss and cell-cell uncoupling. It is thought this MoA has an additional and important structural role for maintaining BRB integrity beyond the classical Gap Junction intercellular communication (GJIC) signalling role with small ions (e.g. $Ca^{2+}$) and other small molecules when intercellular coordination in certain tissues is critically important (e.g. cardiomyocytes of the heart). This MoA is up-stream of the leading anti-VEGF therapies and works at the core pathology in DR, DME, and AMD. Therefore, the compounds described herein, such as danegaptide, may be useful in the prevention of the development of such diseases, as well as useful in the treatment of such diseases once developed.

The compounds described herein are Gap Junction modulators. Danegaptide described herein is the compound (2S,4R)-1-(2-aminoacetyl)-4-benzoylamino-pyrrolidine-2-carboxylic acid (Butera *et al.,* 2009).

Accordingly, in a first aspect, the present invention provides a compound of formula (I):

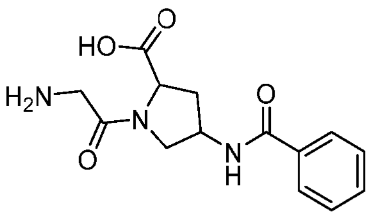 (I),

or a pharmaceutically acceptable salt or hydrate thereof,
for use in a method for the treatment or prevention of age-related macular degeneration (AMD) in a human subject, the method comprising administering to the subject a therapeutically effective amount of the compound; or a pharmaceutically acceptable salt thereof.

Preferably, the compound is (2S,4R)-1-(2-aminoacetyl)-4-benzoylamino-pyrrolidine-2-carboxylic acid; or a pharmaceutically acceptable salt or hydrate thereof.

In some embodiments, the compound or a pharmaceutically acceptable salt or hydrate thereof is administered locally to the eye. In some embodiments, the compound or a pharmaceutically acceptable salt or hydrate thereof is administered locally via intravitreal injection in the eye.

In some embodiments, the compound or a pharmaceutically acceptable salt or hydrate thereof is administered systemically. In some embodiments, the compound or a pharmaceutically acceptable salt or hydrate thereof is administered systemically via oral, subcutaneous, transdermal, or intravenous administration. Preferably, the compound or a pharmaceutically acceptable salt or hydrate thereof is administered systemically via oral administration.

In some embodiments, the method is for the prevention of AMD in a human subject. In some embodiments, the method is for the prevention of progression of AMD in a human subject.

Preferably, the compounds are for use in the method of prevention of progression of dry AMD to wet AMD in a human subject. Preferably, the method is for the prevention of progression of intermediate dry AMD to advanced forms of dry AMD in a human subject. Preferably, the AMD is characterized as early stage dry AMD. Preferably, the AMD is

characterized as intermediate stage dry AMD. Preferably, the AMD is characterized as advanced stage dry AMD. Preferably, the advanced stage dry AMD is geographic atrophy (GA). These embodiments are advantageous because there are no known treatments for dry AMD.

In some embodiments, the AMD is characterized as wet, neovascular, or advanced angiogenic AMD. In some embodiments, the method is for the prevention of development of choroidal neovascularization in a human subject.

In some embodiments, the patient has a comorbidity. For example, in some embodiments, the patient has chronically high blood glucose levels including for a patient that is brought into glycaemic control. In some embodiments, the patient also has type 1 diabetes or type 2 diabetes.

In some embodiments, the patient also has hypertension or chronically high blood pressure including for a patient that is brought into normal blood pressure control. In some embodiments, the patient is brought into normal blood pressure control by hypertensive treatment, glycaemic control, or reductions in cholesterol. In some embodiments, the patient has high cholesterol.

In some embodiments, the patient also has diabetic retinopathy or diabetic macular edema. In some embodiments, the patient has retinal vein occlusion (RVO) eye disease. In some embodiments, the patient has glaucoma disease with or without pathological intraocular pressure. In some embodiments, the patient has Uveitis or other forms of inflammatory eye disease.

In some embodiments, the patient has or had drusen deposits or protein exudates in the eye.

In some embodiments, the compound inhibits dysfunction of the RPE (retinal pigmented epithelium) or the outer blood retina barrier (oBRB), including loss of barrier integrity between the RPE cells and loss of RPE cells. In some embodiments, the compound inhibits stress induced dysfunction of the retinal or choroidal layers in the eye. In some embodiments, the compound inhibits the loss of photoreceptors in the eye, such as the retina. In some embodiments, the compound inhibits vascular leakage in the retina, including vascular leakage leading to macular edema. In some embodiments, the compound improves fluid extraction from the retinal tissues via the retinal pigmented epithelium. In some embodiments, the compound inhibits death or loss of retinal endothelial cells, choroid endothelial cells, pericytes or epithelial cells.

In some embodiments, the compound inhibits pathological hemichannel opening and/or ATP release, such as Connexin 43 mediated opening and/or ATP release. The pathological effects of hemichannel opening and ATP release (or other such small, <1kDa signalling molecules) can be both autocrine and paracrine.

In some embodiments, the concentration of the administered compound, or pharmaceutically acceptable salt, hydrate or formulation thereof, such as an oral, systemic and sustained-release compound formulation, of about 50 nM to about 150 nM, such as about 100 nM, is reached in the microenvironment of the eye.

In some embodiments, the compound is administered to the patient once or twice daily. In some embodiments, the compound is administered to the patient up to four times daily.

In a further aspect, the present invention provides pharmaceutical compositions adapted for the delivery of the compounds to the eye.

The compounds described herein may enable cells to protect themselves during age-related macular degeneration (AMD). Without wishing to be bound to any particular theory, the inventors speculate that the compounds may have stabilizing effects on the cells, reducing the tendency for mitochondria to become leaky and/or reducing the tendency for cells to develop a leaky outer cell membrane and/or improving the intercellular and/or structural coupling of cells. The compounds may result in better coupling between cells, enabling the cells to share available energy (ATP) and enable intercellular signalling, including calcium signalling. The compounds may also result in reduced pathological stress-induced ATP leakage into the extracellular compartment leading to inflammation. The compounds may also functionally improve barrier integrity of the iBRB and oBRB and/or improve the fluid absorptive capacity of the RPE.

In a further aspect, the present invention provides the use of a compound of formula (I):

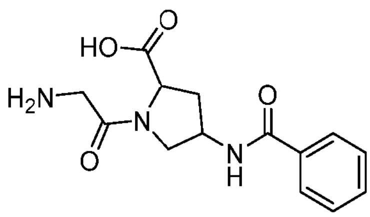

(I),

or a pharmaceutically acceptable salt or hydrate thereof,
for the preparation of a medicament for the treatment or prevention of age-related macular degeneration (AMD) in a human subject, the method comprising administering to the subject a therapeutically effective amount of the compound, or a pharmaceutically acceptable salt or hydrate thereof.

In a further aspect, the present invention provides a method of treating or preventing age-related macular degeneration (AMD) in a human subject, the method comprising administering to the human subject a therapeutically effective amount of the compound, or a pharmaceutically acceptable salt or hydrate thereof,
wherein the compound a compound of formula (I):

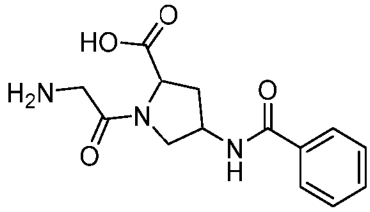
(I),

or a pharmaceutically acceptable salt or hydrate thereof.

In a further aspect, the present invention provides a pharmaceutical composition comprising a compound as described herein for use in a method as described herein, the pharmaceutical composition comprising said compound and a pharmaceutically acceptable excipient. Preferably, the compound is for administration via systemic administration, oral administration, subcutaneous administration, transdermal administration, nasal spray, eye drops, or contact lenses in an eye drop formulation, via a contact lens, via a nasal spray or via injection, e.g. intravitreal injection.

Embodiments of the present invention will now be described by way of example and not limitation with reference to the accompanying figures. However, various further aspects and embodiments of the present invention will be apparent to those skilled in the art in view of the present disclosure.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set outlined above are not limited to any particular aspect or embodiment of the invention and apply equally to all aspects and embodiments which are described.

## Brief Description of the Figures

**Figure 1. Danegaptide protects against DR and AMD-mimicking insult mediated as a decrease in permeability in human retinal pigment epithelial cell monolayers subjected to stress.** Human RPE (retinal pigmented epithelial) cell line, ARPE-19, grown into confluent cell monolayers, showed increases in permeability following 48 h of oxidative damage with sublethal *t*BHP (*tert*-butyl hydroperoxide) in high-glucose conditions that was prevented by danegaptide treatment using two different permeability markers (6-CF and RhoB). Data are presented as mean ± S.E.M. with each data point presenting a separate experimental condition and analyzed using One-Way ANOVA with Holm-Šídák's multiple comparisons test; $P_{app}$ (apparent permeability coefficient), 6-CF (6-carboxyfluorescein; top graph **A**), RhoB (Rhodamine-B; bottom graph **B**), **p* < 0.05, ***p* < 0.01, ****p* < 0.001, n = 6-8.

**Figure 2. Danegaptide protects against DR and AMD-mimicking insult mediated via an improved tight junction organization rate (TiJOR) and improved cell-cell coupling between human retinal pigment epithelial cells in monolayers subjected to stress**. Human RPE (retinal pigmented epithelial) cell line, ARPE-19, grown into confluent cell monolayers showed increases in cell-cell uncoupling and tight junction (TJ) disorganization as measured by immunocytochemistry using anti-zonula occludens- 1 (ZO-1) immunostaining (bottom images **B**; left = control; middle = high glucose and 200 μM *t*BHP; right = high glucose, 200 μM *t*BHP, and 100 nM danegaptide), and the tight junction organization index (TiJOR; top bar graph **A**) following 48 h of oxidative damage with sublethal *t*BHP (*tert*-butyl hydroperoxide) in high-glucose was reduced under cellular stress, which could be prevented by danegaptide treatment. Data are presented as mean ± S.E.M. with each data point presenting a separate TiJOR analysis and analyzed using One-Way ANOVA with Holm-Šídák's multiple comparisons test; TiJOR (the tight junction organization index); HG (hyperglycemia); *t*BHP (*tert*-butyl hydroperoxide); DGP (Danegaptide), ****p* < 0.001, n = 13-16.

**Figure 3. A. Blood glucose levels (mmol/l) and B. blood glycated hemoglobin (HbA1c) levels (mmol/mol) during the *in vivo* studies.** Data are presented as mean ± SD from 15-18 rats per group. Data were analyzed by Two-Way ANOVA (blood glucose, *p* < 0.001; and blood HbA1c, *p* < 0.001) followed by Tukey's post hoc test vs. the Naïve group. ****p* < 0.001.

**Figure 4. Cataract scores during the *in vivo* studies**. Cataracts were scored from 0 (normal lens) to 3 (severe opacities covering more than 75% of the lens). Data are presented as mean ± S.E.M. from 15-18 rats per group. Data were analyzed by Two-Way ANOVA ($p < 0.001$) followed by Tukey's post hoc test vs. the Naïve group. ***$p < 0.001$.

**Figure 5. Danegaptide protects against outer retinal thickening in rats with DR after Streptozotocin (STZ) induction**. Danegaptide prevented thickening in the outer blood retina barrier, which measured outer plexiform and nuclear layers, inner and outer segments and RPE/choroid layers using Spectral Domain Optical Coherence Tomography (SD-OCT) imaging. The SD-OCT retinal scans were segmented, and the thickness of the outer retinal layer was analyzed by a convolutional neural network algorithm. A. STZ-induction in Brown Norway rats resulted in a statistically significant increase in outer retinal thickness in STZ + Vehicle (n = 21) retina after 9 weeks of induction when compared to naïve (n = 16; t-test $p < 0.01$). **B**. Danegaptide treatment by localized eye injections targeting 100 nM every week for three weeks prevented this increase in outer retinal thickness ($p < 0.05$), while anti-VEGF and anti-VEGF + Danegaptide IVT injection treatments had no effect. Data are presented as mean ± S.E.M. *$p < 0.05$; **$p < 0.01$. **C**. Representative SD-OCT scans of outer retinal thickness from the left and right eye nine weeks after STZ-induction.

Figure 6. Schematic drawing indicating the areas used for the region analysis of retinal thickness. ST: superior temporal; SN, superior nasal; IT, inferior temporal; IN, inferior nasal.

**Figure 7. Danegaptide protects against outer retinal thickening throughout the retina. A-D**. Outer retinal thickness was statistically significantly increased in STZ + Vehicle-treated rats compared with naïve rats in all regions, Superior-Temporal (**A**), Superior-Nasal (**B**), Inferior-Temporal (**C**), and Inferior-Nasal (**D**). Data were analyzed by unpaired t-test. **E-H.** Danegaptide prevented thickening of the outer retina in all areas, Superior-Temporal (**E**), Superior-Nasal (**F**), Inferior-Temporal (**G**), and Inferior-Nasal (**H**), as determined by One-Way ANOVA or Kruskal-Wallis ($p < 0.01$), followed by Tukey's or Dunn's multiple comparisons test. Data are presented as mean ± S.E.M. from n = 12-18 rats per group. *$p < 0.05$; **$p < 0.01$; ***$p < 0.001$.

**Figure 8. Regional analysis of inner retinal thickness.** While STZ-induced animals presented with thinner retinas compared to naïve animals (**A-D**), no statistically significant differences were identified between treatment groups (**E-H**). Superior-Temporal (**A** and **E**), Superior-Nasal (**B** and **F**), Inferior-Temporal (**C** and **G**), and Inferior-Nasal (**D** and **H**). Data are presented as mean ± SD from 12-18 rats per group. Naïve and STZ + Vehicle data were analyzed by unpaired t-test. *$p < 0.05$; **$p < 0.01$; ***$p < 0.001$. STZ treatment groups data were analyzed by one-way ANOVA or Kruskal-Wallis ANOVA and $p$ values were >0.05 for all regions.

**Figure 9. Danegaptide and anti-VEGF treatment reduce Evans Blue (EB) extravasation in the diabetic retina. A.** Induction of hyperglycemia by STZ resulted in a significant 6.28-fold increase in EB extravasation 9 weeks after induction (100 ± 13.5% in Naïve vs. 628 ± 212.5% in STZ + Vehicle, n = 10-11, t-test $p < 0.01$). **B**. Anti-VEGF ($p < 0.01$), Danegaptide ($p < 0.05$) and the combination treatment ($p < 0.05$) resulted in statistically significant reductions of EB extravasation of 82.3%, 68.9% and 65.6%, respectively. Data from STZ-induced groups passed normality using a D'Agostino & Pearson test and were analyzed by One-Way ANOVA ($p < 0.01$) followed by Holm-Šídák's multiple comparisons test. Data are shown as mean ± S.E.M. *$p < 0.05$, **$p < 0.01$, ***$p < 0.001$.

**Figure 10. Danegaptide prevents pericyte loss 9 weeks after induction of STZ. A.** Induction of DR by STZ resulted in a statistically significant increase in pericyte ghosts after 9 weeks (t-test, $p < 0.05$, n = 45-67). **B**. The number of pericyte ghosts in the STZ groups was compared using One-Way ANOVA ($p < 0.001$), followed by Dunnett's multiple comparisons test. Both AF564 ($p < 0.01$, n = 45) and Danegaptide ($p < 0.001$, n = 58) resulted in a statistically significant reduction of pericyte ghosts. The combination treatment of AF564 and Danegaptide had no effect on the presence of pericyte ghosts ($p = 0.11$; n = 61). Data are presented as mean ± S.E.M. *$p < 0.05$, **$p < 0.01$, ***$p < 0.001$.

**Figure 11. Representative images from trypsin digests showing protection from pericyte loss by Danegaptide.** PAS staining was used to evaluate vascular abnormalities, including acellular capillaries (arrows) and pericytes ghost (arrowheads). Pericytes are not indicated. Scale bar = 100 µm.

## Detailed Description

### Definitions

Unless specified otherwise, the following definitions are provided for specific terms.

In the present description and claims the standard three-letter and one-letter codes for natural amino acids are used. The term "peptide" herein designates a chain of two or more amino acid moieties (amino acid residues) that are linked by means of a peptide bond. In general, peptides may contain one or more naturally occurring amino acids and/or one or more non-naturally occurring amino acids.

In the present context, the term "naturally occurring amino acid" refers to one of the following 20 amino acids: Ala (A), Cys (C), Ser (S), Thr (T), Asp (D), Glu (E), Asn (N), Gln (Q), His (H), Arg (R), Lys (K), Ile (I), Leu (L), Met (M), Val (V), Phe (F), Tyr (Y), Trp (W), Gly (G), and Pro (P). In naturally occurring peptide molecules these amino acids (with the exception of Gly, which lacks a chiral centre) generally occur in the form of L-amino acid residues, but compounds suitable for use in the present invention include peptides comprising D-amino acid residues.

Amino acid three letter codes are as used in the art. Hyp refers to 4-hydroxyproline.

The compounds for use in the present invention may contain two or more asymmetric atoms (also referred to as chiral centres), giving rise to the possibility of the occurrence of diastereomers. Compounds suited for use in the present invention include such diastereomers.

As used herein, the term "vascular cells" includes endothelial cells and pericytes, including retinal endothelial cells and retinal choroid endothelial cells.

<u>Age-related Macular Degeneration (AMD)</u>

AMD is a degenerative disorder of the central retina (where the central vision is processed in the area known as the macula). The macula is important for the sharp, straight-ahead vision that is used for reading, recognizing faces, and driving. AMD can be classified as dry AMD (d-AMD; also known as non-neovascular AMD or non-exudative AMD) or neovascular AMD (n-AMD; also known as wet AMD or exudative AMD).

Typically, wet AMD usually begins as the dry type of AMD. The typical stages of AMD are:

**1. Early Stage dry AMD.** Characterized by medium drusen (from 63 to 125 microns or μm) but without pigmentary changes or abnormalities (Ferris *et al.,* 2013). This stage is also termed early AMD.

**2. Intermediate Stage dry AMD.** Characterized by either large drusen (more than 125 microns or μm) or pigmentary changes/abnormalities with at least medium drusen. This stage is also termed intermediate AMD.

**3. Advanced Stage dry AMD.** Characterized by lesions associated with tissue atrophy and/ or progressive development of atrophic areas in or near the macular. This stage of d-AMD includes geographic atrophy (GA), characterized by atrophy of outer retinal tissue, retinal pigment epithelium, photoreceptors and/ or choriocapillaris. This stage is also termed late AMD. Geographic atrophy (GA) is a chronic progressive degeneration of the macula, as part of late-stage age-related macular degeneration (AMD). The disease is characterized by localized sharply demarcated atrophy of outer retinal tissue, retinal pigment epithelium and choriocapillaris.

**4. Wet or Neovascular or Advanced Angiogenic AMD.** Characterized by choroidal neovascularization (CNV). Neovascularization is new blood vessel growth or angiogenesis. These new vessels are immature, fragile and leak fluid and blood easily. They also can create scar tissue and as a result, reduce vision or cause retinal detachment. Wet AMD refers to a component of leaky vessels, where excessive vascular leakage may result in formation of retinal edema. New vessel formation can occur with or without substantial edema, and edema can occur without substantial neovascularization or angiogenesis. These disease stages are also termed exudative AMD and late AMD.

As the present invention targets the role of Gap Junctions, in preferred aspects, the present invention is concerned with the treatment of patients with AMD, i.e. stages 1 to 4 above, more preferably stages 1 to 3 above (i.e. dry AMD), or as a therapy which can prevent further worsening of AMD towards more advanced or late disease stages. The invention is also concerned with other vascular stress factors such as a preventative therapy for patients having diabetes who are at risk of developing AMD.

The diabetic patients who are at risk of developing AMD include those with all types of diabetes (type 1, type 2, and gestational). The risk of developing AMD increases the longer a person has diabetes. Accordingly, the present invention may be used for the treatment of all of these patient types. Although the pathophysiological link between diabetes and AMD is not fully understood, the high glucose stressed and more leaky blood vessels observed in diabetes may lead to increased drusen deposition, ROS and hypoxic conditions, and VEGF is a common and important pathological growth factor in both diseases.

In addition, the medical uses and methods of the present invention may be employed for the preventative use in patients being brought into glycaemic control. In such treatment, when a patient with chronically high blood glucose levels is brought into glycaemic control, the eye tissues may have become used to the high glucose availability and become

stressed as normal glucose levels are achieved. These patients are at risk of experiencing worsening of AMD and could be protected as blood glucose is being brought under control. Accordingly, the present invention may be used to as a protective treatment as patients are being brought into glycaemic control.

Diabetic retinopathy (DR) involves changes to retinal blood vessels that can cause them to bleed or leak fluid, distorting vision. Diabetic retinopathy is the most common cause of vision loss among people with diabetes and a leading cause of blindness among working-age adults. Diabetic macular edema is a consequence of diabetic retinopathy that causes swelling in the macula region of the retina.

Diabetic macular edema (DME) is the build-up of fluid (edema) in the macula. DME is the most common cause of vision loss among people with diabetic retinopathy. About half of all people with diabetic retinopathy will develop DME at some point in their life. Although it is more likely to occur as diabetic retinopathy worsens, DME can happen at any stage of the disease.

Patients with diabetic retinopathy and/or diabetic macular edema are also at risk of developing AMD. As described above, patients with diabetes have an increased risk of developing AMD, and this risk is further increased if the patient also develops diabetic retinopathy or diabetic macular edema. In both conditions the inner and outer blood-retinal barriers are being challenged due to pathological stressed conditions and become leaky. Accordingly, the present invention may be used to as a treatment or prevention in patients who also have diabetic retinopathy or diabetic macular edema.

Retinal vein occlusion (RVO) is where a blockage in an artery or vein (called an occlusion or stroke) occurs in the artery or vein controlling the blood flow to and from the retina. This typically occurs when the blood clot is blocking the retinal vein. Patients with retinal vein occlusion (RVO) are also at risk of developing AMD. The ischemic conditions that result from RVO is known to drive pathologically high VEGF levels, and the elevated VEGF levels in the eye can stimulate or worsen AMD development, including neovascular AMD or wet AMD. The highly elevated VEGF levels observed in the eye of a patient with RVO is known to cause significant vascular leakage and formation of edema, and here it would be beneficial to dampen the vascular leakage and strengthen both the iBRB and the oBRB as disclosed herein using a Gap Junction modulating agent. Accordingly, the present invention may be used to as a treatment or prevention in patients who also have retinal vein occlusion (RVO) eye disease.

Glaucoma is a common eye condition where the optic nerve, which connects the eye to the brain, becomes damaged. It is usually caused by fluid building up in the front part of the eye, which increases pressure inside the eye. However, glaucoma can occur without the increase in intraocular pressure. Glaucoma can lead to loss of vision if it is not diagnosed and treated early. Patients with glaucoma disease, both with or without pathological intraocular pressure, are at risk of developing AMD. The retinal pigmented epithelium (RPE) serves an important role of removing fluid from the retina and from the eye in general. If fluid drainage from the anterior part of the eye is reduced, as often observed in glaucoma, the removal of fluid from the posterior segment of the eye via the RPE becomes increasingly important to avoid increased ocular pressure. Hence, therapeutic interventions, such as the compounds described herein including danegaptide, that can stabilize the coupling of RPE cells and improve their function, including fluid removal from the eye are beneficial for patients with glaucoma and patients with glaucoma at risk of developing AMD. Additionally, the neuronal tissue and the optic nerve is observed to be stressed in patients with glaucoma and with a risk of atrophy of the neuronal tissue developing over time. Again, here a therapeutic intervention such as the compounds described herein including danegaptide that can protect the RPE and the outer blood-retinal barrier (oBRB) will help the functional and metabolic support which the RPEs deliver to the neuronal tissues, including photoreceptors, rods, and cones. Accordingly, the present invention may be used to as a treatment or prevention in patients who also have glaucoma disease with or without pathological intraocular pressure.

Inflammatory eye diseases, such as uvetis, occurs in response to infection, allergies, autoimmune disorders, irritation, injury, or trauma to the eyes, eyelids, or surrounding tissues. Uvetis is where the inflammation occurs in the middle layer of tissue in the eye wall (uvea). Patients with uveitis or other forms of inflammatory eye disease are at risk of developing AMD. The inflammatory conditions as observed in uveitis are known to stimulate pathologically elevated levels of several cytokines and growth factors, including VEGF. These signalling molecules can stress the RPE further and reduce the barrier integrity and function of the RPE, and thus, outer retinal layer. Additionally, it is known that pathological stress on the RPE can lead to Cx43 based hemichannel opening and excessive ATP leakage out to the extracellular matrix, where ATP is responsible for purinergic receptor stimulation, which again has a leading role in inflammation and angiogenesis (Clapp *et al.,* 2019). Hence, therapeutic interventions, such as the compounds described herein including danegaptide, that can stabilize the intercellular coupling of RPE cells and prevent pathological opening of Cx43 based hemichannels are beneficial for patients with uveitis and patients with uveitis at risk of developing AMD. Accordingly, the present invention may be used to as a treatment or prevention in patients who also have uveitis or other forms of inflammatory eye disease.

Drusen is characteristic of macular degeneration. Drusen are tiny yellow or white accumulations of extracellular material that build up between Bruch's membrane and the retinal pigment epithelium (RPE) of the eye. The drusen contain proteins and lipids (naturally occurring molecules that include fats). Patients with drusen deposits or other protein

exudates in the eye are at risk of developing AMD. The deposition of drusen reduces the effectiveness of the exchange of oxygen, nutrients and waste products between the RPE and the retinal choroid plexus. Given that the retina and the photoreceptors have a very high energy consumption and a need for effective oxygenation, any reduction in the RPE's ability to support the photoreceptors and neuronal tissue can result in stress of the retinal tissues and increased risk of developing AMD. Hence, a therapeutic intervention, such as the compounds described herein including danegaptide, that can stabilize the RPE and improve the metabolic function and exchange of oxygen, nutrients, and waste products between the RPE and the retinal choroid plexus would be beneficial for patients with drusen or being at risk of increased drusen deposition, such as drusen deposition as observed in AMD. Accordingly, the present invention may be used to as a treatment or prevention in patients who also have drusen deposits or protein exudates in the eye or are at risk of increased drusen deposition.

Various routes of administration may be employed in connection with the methodology of the present invention, including, but not limited to, intraocular injection, systemic administration, oral administration, subcutaneous administration, transdermal administration, nasal spray, eye drops, or contact lenses. In some embodiments, the compound or a pharmaceutically acceptable salt or hydrate thereof is in a drug formulation administered locally (e.g. intravitreal injection) in the eye or systemically (e.g. oral, subcutaneous, transdermal, or intravenous administration) for therapeutic uses thereof. Preferably, the compound or a pharmaceutically acceptable salt or hydrate thereof is administered systemically via oral administration.

In this connection, attainment of a concentration of the administered compound (or pharmaceutically acceptable salt or hydrate thereof) in the plasma of the subject in the range of from 50 nM to 5 μM may be desirable. The *in vitro* studies provided herein showed that for RPE cells an optimal effect was observed when target concentrations of 100 nM of the Gap Junction modulator compounds were reached in the microenvironment of the RPE cell cultures. Additionally, the *in vivo* studies performed in diabetic rats supported that IVT injections of danegaptide at doses that were selected to reach 100 nM in the retinal tissues also conferred protective effects of the outer retinal layers, including the RPE. By way of example, these therapeutic and protective effects may be achieved when a plasma concentration of 50-150 nM was targeted by osmotic pump, or by local injection into the eye targeting a higher concentration in *corpus vitreum,* allowing for a concentration gradient towards the retinal vessels. These data teach that effective doses to be administered to reach 50-150 nM in the microenvironment of the retinal tissues, including the RPE.

Compounds suited for use in accordance with the invention

An example of a compound well suited for use in accordance with the present invention is 1-(2-aminoacetyl)-4-benzoylamino-pyrrolidine-2-carboxylic acid, such as the (2S,4R) diastereomer thereof [i.e. (2S,4R)-1-(2-aminoacetyl)-4-benzoylamino-pyrrolidine-2-carboxylic acid], or a pharmaceutically acceptable salt or hydrate thereof. An example of an alternative name for this compound is (2S,4R)-1-(2-aminoacetyl)-4-benzamidopyrrolidine-2-carboxylic acid.

Other diastereomers of the latter compound (i.e. the 2S4S, 2R4R, 2S4R or 2R4S diastereomers) may also be value for use in the context of the present invention.

Particular forms of this compound may also be referred to as danegaptide.

Pharmaceutically acceptable salt of danegaptide include danegaptide hydrochloride.

The compounds described herein are Gap Junction-dependent cellular modulators. In some embodiments, the compounds may inhibit the dysfunction of the RPE (retinal pigmented epithelium) barrier, including loss of barrier integrity between the RPE cells and loss of RPE cells. In some embodiments, the compound may inhibit dysfunction or the retinal or choroidal layers in the eye. In some embodiments, the compound may inhibit the loss of photoreceptors in the retina. In some embodiments, the compound may inhibit vascular leakage in the retina, including vascular leakage leading to macular edema. In some embodiments, the compound may improve fluid resorption from the retinal tissues by improving barrier integrity and function of the RPE, thereby helping to treat and reduce macular edema. In some embodiments, the compound may inhibit the death or loss of retinal endothelial cells, choroid endothelial cells, pericytes or epithelial cells, including RPE cells. In some embodiments, the compound may inhibit pathological hemichannel opening and/or ATP release, such as Connexin 43 mediated hemichannel opening and/or ATP release. The pathological effects of hemichannel opening and/or ATP release (or other such small signalling molecules) can be both autocrine and paracrine.

For example, Squires *et al.,* 2021 have shown that danegaptide can reduce ATP leakage from kidney epithelial cells. Mugisho *et al.,* 2019 showed Peptide5, a Connexin 43 hemichannel blocker, decreased inflammatory cytokine and ATP release which may be useful in treating inflammatory diseases, such as AMD and DR. González-Casanova *et al.,* 2021 suggested connexin hemichannel blockers or drugs to enhance Gap Junction intercellular communication (GJIC) may be useful in treating DR. Subauste, 2019 studied the CD40-ATP-$P2X_7$ receptor pathway and linked it to induction of inflammatory responses and endothelial cell death. Clapp *et al.,* 2019 suggested the protective role of blocking the $P2X_7$ receptor in DR and/or AMD may be due to the inhibition of inflammatory cytokine release and the inhibition of VEGF release.

A review by King *et al.* (2021) disclosed different Cx43 targeting molecules, however these molecules did not

block hemichannels and stabilize Gap Junctions under stressed conditions in AMD simultaneously. In contrast, the compounds disclosed herein, including danegaptide, have a competitive advantage of not only blocking hemichannels but also effectively stabilizing Gap Junction-dependent intercellular coupling under stressed conditions in AMD.

In addition to the compounds recited above, further compounds that may be suitable for use in the context of the invention include certain other Gap Junction modulating compounds, such as the antiarrhythmic peptides AAP (Aonuma *et al.,* 1980), AAP10 (Dhein *et al.,* 1994; Muller *et al.,* 1997), HP5 (disclosed in U.S. Patent No. 4,775,743) and other antiarrhythmic peptides as disclosed in WO 02/077017, WO 2007/078990, or WO 2018/202865.

It will be appreciated that the compounds described herein may be used in combination. For example, more than one Gap Junction modulator compound may be administered in the methods described herein, either concurrently or sequentially.

Pharmaceutically Acceptable Salts

Pharmaceutically acceptable salts of compounds suited for use in accordance with the invention having an acidic moiety can be formed using organic or inorganic bases. Suitable salts formed with bases include metal salts, such as alkali metal or alkaline earth metal salts, for example sodium, potassium, or magnesium salts; ammonia salts and organic amine salts, such as those formed with morpholine, thiomorpholine, piperidine, pyrrolidine, a mono-, di- or tri-lower alkylamine *(*e.g., ethyl-tert-butyl-, diethyl-, diisopropyl-, triethyl-, tributyl- or dimethylpropylamine), or a mono-, di- or trihydroxy lower alkylamine (*e.g.,* mono-, di- or triethanolamine). Internal salts also can be formed. When a compound suited for use in accordance with the invention contains a basic moiety (as in the case of, e.g., 1-(2-aminoacetyl)-4-benzoylamino-pyrrolidine-2-carboxylic acid and the recited diastereomers thereof), salts may be formed using organic or inorganic acids. For example, salts can be formed from the following acids: acetic, propionic, lactic, citric, tartaric, succinic, fumaric, maleic, malonic, mandelic, malic, phthalic, hydrochloric, hydrobromic, phosphoric, nitric, sulfuric, methanesulfonic, napthalenesulfonic, benzenesulfonic, toluenesulfonic or camphorsulfonic. Other known pharmaceutically acceptable acids may also be employed. As already mentioned (*vide supra*), a preferred salt form of the (2S,4R) diastereomer of 1-(2-aminoacetyl)-4-benzoylamino-pyrrolidine-2-carboxylic acid is the hydrochloride monohydrate.

The present teachings may also extend to the use of prodrugs of the compounds disclosed herein as being suited for use in accordance with the present invention. As used herein, "prodrug" refers to a moiety that produces, generates or releases a compound of one of the disclosed types when administered to a mammalian subject, notably a human subject. Prodrugs can be prepared by modifying functional groups present in the compounds in such a way that the modifications are cleaved, either by routine manipulation or *in vivo,* from the parent compounds. Examples of prodrugs include compounds as disclosed herein that contain one or more molecular moieties appended (bonded) to a hydroxy, amino, sulfhydryl or carboxy group of the compound, and that when administered to a subject to be treated are cleaved *in vivo* to form the free hydroxy, amino, sulfhydryl or carboxy group, respectively. Examples of prodrugs include, but are not limited to, acetate, formate and benzoate derivatives of alcohol and amine functional groups in the compounds disclosed herein for use in accordance with the invention. Examples of preferred prodrugs include oxazolidinone or imidazolidinone prodrugs. Ester prodrugs may be formed with lower alcohols, such as $C_{1-6}$ alcohols. Preparation and use of prodrugs is discussed in T. Higuchi and V. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987.

Pharmaceutical Compositions

Compounds, or pharmaceutically acceptable salts or hydrates thereof, employed in accordance with the present invention may be administered in the form of appropriate pharmaceutical compositions, which can be administered via any acceptable method known in the art, either singly or in combination. Pharmaceutical compositions of relevance in the present context may comprise a compound as disclosed herein for use in accordance with the invention in admixture with one or more pharmaceutically acceptable carriers, diluents, vehicles, or excipients. In general, the pharmaceutical compositions used in accordance with the present invention may be adapted for administration of the compound in eye drops, contact lenses, nasal spray, intravitreally or systemically.

Useful formulations may include formulations that provide sustained release of the compounds of the present teachings. These may be particularly useful for subsequent administration (after the first administration). The compositions are preferably in the form of liquid formulations, and methods for their preparation are generally described in "Remington's Pharmaceutical Sciences", 17th Ed., Alfonso R. Gennaro (Ed.), Mark Publishing Company, Easton, PA, U.S.A., 1985. Such compositions generally contain an effective amount of the one or more active compounds of the present teachings, together with a suitable carrier in order to provide the dosage in a form compatible with the route of administration selected. Preferably, the carrier is in the form of a vehicle, a diluent, a buffering agent, a tonicity adjusting agent, a preservative, inhibitor, and/or a stabilizer. The excipients constituting the carrier must be compatible with the

active pharmaceutical ingredient(s) and are preferably capable of stabilizing the compounds without being deleterious to the subject being treated.

A form of repository or sustained-release formulation can be used so that therapeutically effective amounts of the preparation are delivered into the bloodstream over many hours or days following administration of the compound or composition, e.g., by transdermal injection or deposition. Formulations suitable for sustained release may comprise biodegradable polymers, such as L-lactic acid, D-lactic acid, DL-lactic acid, glycolide, glycolic acid, and isomers thereof. Similarly, the carrier or diluent can include any sustained release material known in the art, such as glyceryl monostearate or glyceryl distearate, alone or mixed with a wax.

Other sustained release formulations can include, but are not limited to, formulations that include at least one of the compounds disclosed herein combined with liposomes, microspheres, emulsions or micelles and liquid stabilizers.

Administration of a compound (or pharmaceutical salt or hydrate thereof) in accordance with the invention may be conducted in a single unit dosage form (e.g. in the form of a bolus) or as a continuous therapy in the form of multiple doses over time. Alternatively, continuous infusion systems or slow-release depot formulations may be employed. Two or more compounds for use in accordance with the invention (or pharmaceutical compositions thereof) may be co-administered simultaneously or sequentially in any order. In addition, the compounds and compositions may be administered in a similar manner for prophylactic purposes, for example if a diabetic patient or a patients with drusen deposits deemed to be at risk of developing AMD or worsening of AMD or macular edema associated with AMD. Ultimately, the best dosing regimen will be decided by the attending physician for each patient individually.

## Therapeutic Uses

Conditions which may be treated or prevented in accordance with the present invention using compounds as specified herein include age-related macular degeneration (AMD), especially in a human subject.

According to the invention, one or more of the compounds, or pharmaceutically acceptable salts or hydrates thereof (e.g. in the form of an appropriate pharmaceutical composition), may be administered to an individual in need thereof in a therapeutically effective amount.

As used herein, "a therapeutically effective amount" refers to an amount that is capable of reducing the symptoms of a given neurovascular condition or pathology in the eye, and preferably which is capable of partly or wholly normalizing physiological responses in a subject with the condition or pathology. Reduction of symptoms or normalization of physiological responses can be determined using methods known in the art and can vary with a given condition or pathology. The effective amount will be determined by the skilled person taking into account such factors as potency of the drug, age and constitution of the patient, body weight, pharmacokinetic profile of the drug, and in general the drug will be prescribed for each patient or group of patients.

The effective amount of the compound can be at least about 10 μg/kg body weight/day, such as at least about 100 μg/kg body weight/day, at least about 300 μg/body weight/day, and at least about 1000 μg/kg body weight/day. On the other hand, the effective amount of the compound or dimer can be at most about 100 mg/kg body weight/day, such as at most about 50 mg/kg body weight/day and at most about 10 mg/kg body weight/day. It is expected that the effective amount of the compound will be about 100 μg/kg body weight/day, about 300 μg/kg body weight/day or about 1000 μg/kg body weight.

The experiments provided in the *in vitro* system described herein dose findings as performed in the *in vitro* system (cell coupling assay/SLDT) show that a concentration of 50-150 nM is optimal in the microenvironment, and the *in vivo* pilot study showed that 1000 nM injections were better than 200 nM injections when targeting 100 nM in the microenvironment of the eye.

In some embodiments, the compound is administered to the patient once or twice daily, that is sometimes referred to as QD (*quaque die*) or BID (*bis in die*), respectively. In some embodiments wherein the compound is administered to the patient twice daily (BID), the dosage is about 75 mg/kg per administration. In some embodiments, the compound is administered to the patient four times daily, that is sometimes referred to as QID (*quater in die*). In some embodiments wherein the compound is administered to the patient four times daily (QID), the dosage is from about 75 mg/kg to about 125 mg/kg per administration.

## Experimental Examples

### Gap Junction and Hemichannel Modulator Compounds

Compounds (peptides) for use in accordance with the present invention may suitably be synthesized by means of solid-phase or solution-phase synthesis. In this context, reference may be made, for example, to Fields et al., "Principles and practice of solid-phase peptide synthesis", Synthetic Peptides (2002, 2nd Edition).

With regard to the preparation of 1-(2-aminoacetyl)-4-benzoylamino-pyrrolidine-2-carboxylic acid, such as the

(2S,4R) diastereomer thereof, suitable methods of synthesis and purification thereof are described in WO 2007/078990, in which the (2S,4R) isomer is denoted "Compound 2" (WO 2007/078990).

An example of a useful salt form of the (2S,4R) diastereomer is the hydrochloride monohydrate, the preparation of which is described in WO 2008/079266, and which is also referred to in the present description as Compound X (WO 2008/079266).

*In vitro* studies - **Figures 1 and 2**

**Cell line and cell culture**

Human retinal pigment epithelial cells (ARPE-19, CRL-2302™, American Tissue Type Collection, ATTC, Manassas, VA) were used. Cells were passaged and maintained per the supplier's instructions. Briefly, cells were cultured in a 1:1 mixture of Dulbecco's modified Eagles medium and Ham's F12 medium containing 1.2 g/L sodium bicarbonate, 2.5 mM L-glutamine, 15 mM HEPES and 0.5 mM sodium pyruvate and fetal bovine serum to a final concentration of 10% and cultured at 37 °C in an atmosphere of 5% $CO_2$/95% humidity. The cell cultures were supplemented with penicillin/streptomycin to a final concentration of 100 U/ml).

The cells were maintained in T25 and T75 tissue culture flasks (TPP Techno Plastic Products AG, Trasadingen, Switzerland). For experiments, cells were seeded in transwell inserts (150,000-260,000 cells/well; 12 mm Transwell® with 0.4 μm Pore Polyester Membrane Insert, Corning, Corning, NY) or multi-chamber slides (100,000 cells per well; Nunc® Lab-Tek IT 8-well Chamber Slides, Thermo Fisher Scientific, Waltham, MA).

**Permeability studies**

For permeability experiments, ARPE-19 cells were grown to confluency in transwell inserts for 48-72 h. Cells were then be pre-treated with Danegaptide (DGP; 100 nM; dissolved in saline) or vehicle control for 24 h. Subsequently, cells were challenged with a combination of sublethal oxidative stress (250 uM *tert-butyl* hydroperoxide; Millipore Sigma, St. Louis, MO) and hyperglycemia (30 mM glucose; Millipore Sigma, St. Louis, MO) for 48 h in the continued presence of 100 nM DGP or vehicle.

To assess permeability, the apical to basolateral movement of the low- and high-permeability dyes, 6-carboxyfluorescein (6-CF; Thermo Fisher Scientific, Waltham, MA) and Rhodamine B (RhoB; Thermo Fisher Scientific, Waltham, MA), respectively was quantified. To this end, the dyes diluted in Hank's Balanced Salt Solution (HBSS, Corning, Corning, NY) were added to the apical compartment (top-insert, donor chamber; 6-CF: 100 μM, RhoB: 50 μM) of the cells. At regular intervals (10, 20, 30, 45, 60, 90 and 120 min), samples (100 μl) from the basolateral compartment (bottom-well, receiver chamber) were collected. The sampled volume was replaced by fresh buffer.

The cumulative concentration of 6-CF and RhoB was calculated using a standard curve (0-10 μM) established using a microplate reader (Cytation5, Agilent, Santa Clara, CA), taking into account the volume removed and replaced with media in the final calculations. 6-CF was quantified using excitation 490 nm/emission 520 nm, while RhoB was quantified using excitation 533 nm/emission 627 nm. See **Figure 1.**

**Quantification of tight junction organization**

For immunocytochemistry, ARPE-19 cells were grown to confluency in chamber slides for 72 h. Cells were then be pre-treated with Danegaptide (DGP; 100 nM) or vehicle control for 24 h. Subsequently, cells were challenged with a combination of sublethal oxidative stress (250 μM *tert*-butyl hydroperoxide; Millipore Sigma, St. Louis, MO) and hyperglycemia (30 mM glucose; Millipore Sigma, St. Louis, MO) for 48 h, in the continued presence of 100 nM DGP or vehicle.

Cells were fixed with 4% paraformaldehyde for 15 minutes, and subsequently blocked and permeabilized in block solution (10% normal goat serum, 1% bovine serum albumin, 0.5% Triton X-100 in 0.1 M Phosphate buffer pH 7.4). Cells were labelled with anti-ZO-1 antibody (mouse anti-human 70-1, 1 pg/ml, cat # 339100, Invitrogen, Thermo Fisher Scientific, Carlsbad, CA) in antibody solution (3% normal goat serum, 1% bovine serum albumin, 0.5% Triton X-100, 0.05% sodium azide in 0.1 M Phosphate buffer pH 7.4) for 2 h at room temperature. After washing 3x in phosphate buffered saline, cells were labelled with fluorescently-conjugated goat anti-mouse IgG (H+L) cross-adsorbed secondary antibody (AlexaFluor® 488, dilution of 1:2,000) for 1 h at room temperature. Nuclei were co-labelled stained with 0.1 pg/ml 4',6-Diamidino-2-phenylindole dihydrochloride (DAPI, Sigma-Aldrich) and washed with phosphate-buffered saline. Chamber slides were coverslipped using Aqua/Poly-Mount (Polysciences, Inc., Warrington, PA). Images were acquired using a Leica SPE confocal microscope (Leica Microsystems, Buffalo Grove, IL). See **Figure 2.**

**Data Analysis**

The apparent permeability coefficient ($P_{app}$, cm/s) was calculated according to Equation (Ziniauskaité *et al.,* 2019):

$$P_{app} = \frac{\Delta Qr/\Delta t}{A \times C_d}$$

where:

$\Delta Qr/\Delta t$= slope of the linear region of the cumulative amount of the study compound in the receiver chamber versus time plot
$C_d$= initial donor concentration of the study compound
$A$ = surface area

See **Figure 1.**
The tight junction organization rate (TiJOR) was calculated from confocal images, as described previously (Terryn *et al.,* 2013). See **Figure 2.**
Data were plotted and analyzed in Prism 9 (GraphPad, Inc., La Jolla, CA). Data are presented as mean ± S.E.M. with each data point presenting a separate experimental condition. Data were analyzed using One-Way ANOVA, groups analyses, along with the Holm-Šídák multiple comparisons test.

*In vivo* studies - **Figures 3-11**

**Animals**

All animals were treated in accordance with the ARVO Statement for the Use of Animals in Ophthalmic and Vision Research, the EC Directive 2010/63/EU of the European Parliament and of the Council on the Protection of animals used for Scientific Purposes and using protocols approved and monitored by the Animal Experiment Board of Finland (animal license number ESAVI-9520-2020).
For these animal studies, Brown Norway rats (strain: BN/Crl rats; Charles River Laboratories, Germany) aged 9-10 weeks at time of induction were housed in individually ventilated cages with aspen bedding, nesting material (*Populus tremula,* Tapvei® , Estonia OÜ) and polycarbonate red tubes (Datesand group) as enrichment, at a constant temperature (22 ± 1°C), relative humidity (50 ± 10%) and in a dim light-controlled environment (lights on from 7 am to 7 pm) with *ad libitum* access to food (Rat/Mouse maintenance V1534-000, ssniff Spezialdiäten GmbH) and tap water. Experiments started after a one-week quarantine and acclimatization in the vivarium.
The body weight of all animals was monitored at baseline and twice per week throughout the study period.
Animal welfare checks were conducted daily. If an animal was identified with welfare problems, medical care was provided under the guidance of the supervising veterinarian in cooperation with the Study Director.
To prevent excessive weight loss after induction, rats were provided daily wet feed (regular feed hydrated with tap water) and supportive feed (Solid Drink - Diet Bio Cup. Triple A Trading). Animals with more than 15 percent weight loss received extra hydration with daily subcutaneous (s.c.) injections (Ringer-Lactat Animalcare. Ecuphar NV). Paraphimosis and urine retention were treated with lidocaine hydrochloride monohydrate (2%, Xylocain, AstraZeneca). Small skin infections occurred, and were treated with fusidic acid (1%, Isathal®, Dechra).
No other medical treatments were used for the animals during the study.
A total of 111 rats were used in the current study. From these, 28 rats were sacrificed prior to the end point due to various welfare issues, mainly due to severe weight loss (>25% from baseline), as commonly observed in the STZ-treated diabetic Brown Norway rats. Animals were randomized into treatment groups based on glucose levels and ocular health. At the end of the study (week 9), a total of 83 rats were included in the study, distributed across five treatment groups:

**Group 1:** Naïve for tissue sampling (n = 18);
**Group 2:** STZ + Vehicle (n = 15);
**Group 3:** STZ + AF564 (or anti-VEGF) (n = 17);
**Group 4:** STZ + Danegaptide (n = 17);
**Group 5:** STZ + AF564 (or anti-VEGF) + Danegaptide (n = 16).

### Anesthesia and Reversal

For all procedures, rats were anesthetized by subcutaneous injection of a mixture containing ketamine (30 mg/kg; Ketaminol vet 50 mg/ml, Intervet) and medetomidine hydrochloride (0.2 mg/kg; Cepetor vet 1 mg/ml; CP-Pharma Handelsgesellschaft mbH). Anesthesia was reversed by the $\alpha_2$-antagonist for medetomidine, atipamezole (1.0 mg/kg; Revertor™ , 5 mg/mL; CP-Pharma Handelsgesellschaft mbH). All the anesthetic reagents were diluted to working stocks in physiological saline solution to enable dosing. Ringer's Lactate solution was administered during anesthesia recovery to prevent dehydration.

### Diabetic retinopathy induction and blood glucose measurements

Prior to the induction the animals were weighed, and blood glucose measured with a rapid meter (AlphaTRAK 2; Zoetis). Rats were fasted for four hours prior to the induction.

Diabetes was induced in the Brown Norway rats (aged 9-10 weeks) by a single dose of streptozotocin (STZ; 65 mg/kg. Sigma) in 10 mM sodium citrate buffer, pH 4.5. After the STZ injection, rats were placed in their cages with *ad libitum* food and 10% (w/v) sucrose solution in tap water for 48 h. The next morning, 5% glucose (1 ml) was injected intraperitoneally or subcutaneously to all induced rats. Four days after the first STZ injection, the success of the induction was monitored by measuring the blood glucose levels from the saphenous vein. The induction of diabetes was considered successful when the blood glucose values measured by the rapidmeter were equal or higher than 16 mmol/l (288 mg/dl). Animals that were not diabetic based on this criterion 96 h after the first STZ injection received a second STZ injection.

The selected rats were monitored for blood glucose levels and animal health for 6 weeks, and then treated with weekly intravitreal (IVT) injections of vehicle or test compounds for three weeks for a total of 3x IVT eye injections per eye per animal.

Blood glucose levels were monitored weekly during the whole follow-up period. In cases where the blood glucose measurement fell out the range of the rapid meter (>41.7 mmol/l), plasma samples were collected, and analyzed using a colorimetric assay (Rat Glucose Assay Kit, Crystal Chem). See **Figure 3A.**

Plasma samples for glycated hemoglobin (HbA1c) measurements were collected five and nine weeks after induction of DR. HbA1c was measured using a commercial turbidimetric immunoassay (Konelab Prime 60i; Thermo Scientific). See **Figure 3B.**

### Preparation of Test Solutions

#### *Vehicle Solution*

A vehicle solution containing D-Mannitol and trisodium citrate dihydrate was prepared as follows:

| Item | Concentration | Unit | Name | Amount |
|---|---|---|---|---|
| 1 | 50 | mg/ml | D-Mannitol | 50.00 g |
| 2 | 0.71 | mg/ml | Tri Sodium Citrate, Dihydrate | 710 mg |
| 3 | q.s. | - | Sodium Hydroxide | q.s. |
| 4 | q.s. | - | Hydrochloric Acid | q.s. |
| 5 | - | - | Water for Injection | Fill up to 1000 ml* |
| (*) Density: 1.079 g/ml, if of relevance to the preparation procedure. | | | | |

The sterile solution were aliquoted in 15 ml falcon tubes, additional two samples of 0.5 ml were stored in standard amber tubes as a retain samples. All the aliquots were stored protected from light at +4 °C.

#### *Danegaptide Solutions*

A stock solution of 0.3 mg/ml Danegaptide was prepared weekly starting on the first day of dosing as follows.

| Item | Concentration | Unit | Name | Amount |
|---|---|---|---|---|
| 1 | 0.3 | mg/ml | Danegaptide# | |

(continued)

| Item | Concentration | Unit | Name | Amount |
|---|---|---|---|---|
| 2 | - | - | Tri Sodium Citrate, Dihydrate | |
| 3 | q.s. | - | Sodium Hydroxide | q.s. |
| 4 | q.s. | - | Hydrochloric Acid | q.s. |
| 5 | - | - | Water for Injection | Fill up to 20.0 ml |
| (#) provided as HCl-salt/Monohydrate, acceptable weight range [6.94-7.34] mg ~ ±3% (2.8) % from target value | | | | |

Two aliquots of 0.5 ml were stored in standard amber tubes as retain samples. The rest of the solution was used to prepare the dosing solutions for that week (to be administered within two days). The stock solution and the aliquots were stored protected from light at +4 °C.

The stock 0.3 mg/ml Danegaptide solution will be diluted to 9 μg/ml as follows.

| Preparation | |
|---|---|
| 1.00 | ml of Danegaptide stock solution (0.3 mg/ml) |
| 32.3 | ml of Vehicle formulation |
| 33.3 | ml of |

Two aliquots of 0.5 ml were stored in standard amber tubes as a retain samples. The aliquots were stored protected from light at +4 °C. The 9 μg/ml Danegaptide solution was further used to prepare the 0.3 μg/ml working solution as follows, and the leftovers were discarded.

| Preparation | |
|---|---|
| 0.700 | ml of Danegaptide stock solution (9.0 μg/ml) |
| 20.3 | ml of Vehicle formulation |
| 21.0 | ml of Danegaptide 0.3 μg/ml dosing formulation |

Two aliquots of 0.5 ml were stored in standard amber tubes as a retain samples. The aliquots were stored protected from light at +4 °C. The leftovers from the working solution were discarded daily.

The 9 μg/ml Danegaptide solution was further used to prepare the 0.6 μg/ml solution that was used to prepare the treatment for the group "STZ + AF564 + Danegaptide" (see, e.g., **Figures 3B, 4, 5C, 9B, and 10B**), "STZ + anti-VEGF + Danegaptide" (see, e.g., **Figure 5B**), or "STZ + AF564 + Dgp" (see, e.g., **Figures 7E-H, and 8E-H**).

| Preparation | |
|---|---|
| 0.670 | ml of Danegaptide stock solution (9.0 μg/ml) |
| 9.330 | ml of Vehicle formulation |
| 10.0 | ml of Danegaptide 0.6 μg/ml dosing formulation |

Two aliquots of 0.5 ml were stored in standard amber tubes as a retain samples. The aliquots were stored protected from light at +4 °C. The leftovers from the working solution were discarded daily.

***Rat VEGF Antibody (AF564) Solutions***

For administration of anti-VEGF treatment ("STZ + AF564" in **Figures 3B, 4, 5C, 7EH, 8E-H, 9B, and 10B**, or "STZ + anti-VEGF" in **Figure 5B**), the rat anti-VEGF antibody (AF564; R&D Systems) was dissolved at a concentration of 0.675 mg/ml in vehicle solution. One vial of 100 μg antibody was dissolved in 150 μl of vehicle solution to prepare the dosing solution.

For co-administration of Danegaptide and anti-VEGF antibody ("STZ + AF564 + Danegaptide" in **Figures 3B, 4, 5C, 9B, and 10B;** "STZ + anti-VEGF + Danegaptide" in **Figure 5B;** or "STZ + AF564 + Dgp" in **Figures 7E-H, and 8E-H**), one vial of antibody (100 μg) was dissolved in 75 μl of Vehicle to make a 2x AF564 stock solution and subsequently combined with an equal volume of a 2x working stock of Danegaptide (0.6 μg/ml), for a final concentration of 0.675 mg/ml AF564 antibody and 0.3 μg/ml Danegaptide in the dosing solution.

Five microliters of dosing solution were delivered by intravitreal injection to the rat eye. The rat eye contains approximately 20-25 μl of vitreous humor. Thus, delivery of 5 μl test article results in dilution to a final concentration of anti-VEGF antibody in the eye of 0.135-0.1125 mg/ml, similar to the human clinical dose of 0.125 mg/ml, according to Filek *et al.* 2019.

**Intravitreal (IVT) Treatment**

For IVT treatment administration, the animals were anesthetized animals, placed under a stereoscope (Leica Microsystems), and a drop of iodine was applied on the cornea and allowed to spread evenly (Minims Povidone Iodine 5%, Bausch & Lomb). A small incision in the choroid exposing the vitreous chamber was performed using a 30G needle near the limbus. A microsyringe with a 33G needle (Hamilton Bonaduz AG, Bonaduz) attached was used to inject the compounds into the intravitreal space. The test solution was injected into the intravitreal space for 10 s, the needle was kept in place for additional 30 s before being removed to avoid the reflux of the compound. Chloramphenicol ointment was applied after the injection (Oftan Chlora, Santen Oy). The different treatments were administered three times by bilateral intravitreal administration of a volume of 5 μl at weeks six, seven, and eight after STZ induction.

***In vivo* imaging**

The development of the diabetic retinopathy and the associated vascular leakage was followed by spectral-domain optic coherence topography (SD-OCT) at week nine after DR induction. The *in vivo* imaging analysis was performed for a subset of study animals (9-14 per treatment group). In addition to the *in vivo* imaging, the development of diabetic cataracts for all the animals was evaluated at weeks six, seven, eight and nine after induction.

***Spectral Domain Optical Coherence Tomography (SD-OCT)***

Anesthetized rats were imaged with SD-OCT (Envisu R2200. Bioptigen Inc./Leica Microsystems) retinal scan at week nine as shown in **Figure 5C.** The scanned area covers a 2.4 x 2.4 $mm^2$ of the retina centred around the optic nerve. Each scan is composed of 100 B Scans each one composed of 1000 A Scans. SD-OCT was performed on both eyes at week nine after DR induction prior to sacrifice and sampling.

The retinal thickness at week nine was analyzed by a convolutional neural network based on U-net architecture using transfer learning approach. The resulting segmentation masks were used to measure the layer thickness in each pixel-width column, i.e. a-scan, resulting in 1000 measuring points for each layer per one b-scan. Only measuring points that passed the automatic quality control criteria set were used in the calculations. The thickness results were presented as SD-OCT measurements of the inner retina, including the nerve ganglion cell layer, inner plexiform and inner nuclear layers; and the outer retina, including the outer plexiform layer, outer nuclear layer, inner and outer segment, and RPE/choroid. Retinal thickness measurements, such as changes in retinal thickness measurements, were obtained by calculating the mean of all the measurements per scan, or by sub-analysis of the superior-temporal, superior-nasal, inferior-temporal and inferior-nasal region (see **Figure 6** for the schematic drawing indicating these area of the eye). See **Figures 5, 7 and 8** for the results of SD-OCT.

**Animal Sacrifice and Tissue Collection**

No samples were collected from animals found dead or sacrificed prior to the study end point.

***Samples for Retinal Vasculature Analysis***

At endpoint, six rats per treatment group were sacrificed by anesthesia overdose, and transcardially perfused with 0.9% NaCl solution (10ml/min for 3 min, 120S/DV Manual Control Variable Speed Pump, Watson-Marlow Pumps). The eyes (n = 12) were enucleated and the orientation marked and placed in 4% PFS prior to preparation of retinal flat mounts.

### *Evans Blue Retinal Leakage Measurement*

Prior to sacrifice, five rats per treatment group were injected intravenously in the tail vein with 4% Evans Blue solution (100 μl/100 g). After 2 h, plasma samples were collected, and the animals were transcardially perfused with physiological saline solution for 30 min at a flow rate of 10 ml/min (120S/DV Manual Control Variable Speed Pump). The eyes (n = 10) were enucleated, the retinas excised and snap frozen and stored at -80 °C until analysis of Evans Blue extravasation. See **Figure 9** for results.

### Tissue processing

### *PAS Histological Staining and Acellular Capillaries and Pericyte Analysis*

Retinal flat mounts were trypsin-digested to isolate the retinal vasculature and stained using Periodic Acid-Schiff (PAS). Briefly, retinal flat mounts were placed in distilled water overnight and incubated with 4% Trypsin in 0.1M Tris-HCl buffer (pH 7.8) for 1.5 hours at + 37 °C. Samples were carefully washed with distilled water to detach the retinal cell layers from the vasculature. The vascular flat mount were dried on a microscope slide and processed for PAS staining. Samples were oxidized, rinsed with $dH_2O$, placed in Schiff's reagent, washed, counterstained with Hematoxylin, differentiated with acid alcohol, dehydrated and mounted with Depex.

Four to six individual images obtained from a central area, and from a peripheral area of the retinal vasculature were acquired using a Leica Thunder 3D Tissue Imager (Leica Microsystems). The number of acellular capillaries, the number of pericytes and the number of pericyte ghosts were manually counted by an investigator blinded to the experimental group assignment.

The number of data points per group were as follows: naïve (n = 67), STZ + Vehicle (n = 59), STZ+AF564 (n = 45), STZ + Danegaptide (n = 58), STZ + AF564 + Danegaptide (n = 61).

See **Figure 11** for representative PAS images, and **Figure 10** for the results.

### *Evans Blue Retinal Extravasation Measurement*

Retinas were homogenized in formamide in a 1:10 ratio (w/v) and incubated overnight at + 70°. Homogenates were centrifuged at 20,800 x g for 45 min and the supernatant transferred into a clean tube. 30 μl of the supernatant was pipetted as triplicate into a 384-well plate. Absorbance (λ = 620 nm) of the samples was measured using a plate reader (Cytation 3, BioTek Instruments Inc., Winooski, VT) and the concentration of Evans Blue in each sample was calculated against a standard curve of Evans Blue and normalized to tissue weight. See **Figure 9** for results.

### Data Analysis

Quantitative data were graphed, analyzed and presented as mean ± standard deviation (SD) or standard error (SEM), as indicated. Normality was assessed and date were log-transformed to achieve a Gaussian distribution, if necessary. Outliers were identified using the ROUT method with a Q coefficient of 1%. Differences were considered statistically significant at the $p < 0.05$ level. Data were analyzed using GraphPad Prism software (v9.1.2. GraphPad Inc., La Jolla, CA) using appropriate statistical tests as indicated.

<u>Results</u>

The results of the experimental studies are shown in **Figures 1-11.**

**Figure 1: Danegaptide protects against DR and AMD-mimicking insult mediated as decrease in permeability in human retinal pigment epithelial cell monolayers subjected to stress.**

A confluent monolayer of human retinal epithelial cells (ARPE-19) in transwell plates were either treated with vehicle (complete media) or danegaptide (DGP, 100 nM) for 24 h. Subsequently, media was replaced with: (i) vehicle (complete media; **Figures 1A and 1B**, left column "-,-"); (ii) media supplemented 30 mM of high glucose (a hyperglycemia stress) and a sublethal concentration of 200 μM *tert*-butyl hydroperoxide (*t*BHP) (an oxidative stress) (**Figures 1A and 1B**, middle column, "+,-"); or (iii) media supplemented with 30 mM glucose, a sublethal concentration of *tert-butyl* hydroperoxide (*t*BHP, 200 μM), plus 100 nM danegaptide (**Figures 1A and 1B**, right column, "+,+"), and incubated for a period of 48 h.

The combination of 30 mM high glucose (hyperglycemia stress) and oxidative stress caused RPE barrier dysfunction and increases in permeability across the RPE monolayer as shown using two fluorescent permeability

markers, 6-carboxyfluorescein (6-CF; **Figure 1A**) or Rhodamine-B (RhoB; **Figure 1B**). The increase in RPE monolayer permeability due to the hyperglycemic and oxidative stressed conditions, and the protection by danegaptide was measured by taking samples at 10, 20, 30, 45, 60, 90 and 120 min after addition of the permeability markers to the transwell chamber insert and apical side of the RPE monolayer and measuring the efflux to, or amount of permeability marker present in the bottom camber of the transwell plates and the basolateral side of the RPE monolayer.

The cumulative concentration of 6-CF and RhoB present in the bottom chamber was calculated by measuring the amount fluorescent signal based on a standard curve established using a microplate reader (Cytation5, Agilent, Santa Clara, CA). The amount of paracellular permeability was quantitated as the apparent permeability coefficient ($P_{app}$) and calculated by determining the steady-state flux. The DR and AMD-mimicking insult of hyperglycemia and oxidative stress significantly increased the permeability of both 6-CF and RhoB (n = 6-8) across RPE monolayers (**Figures 1A and 1B**, middle column, "+,-"; as compared to the control left column, "-,-"), and treatment with 100 mM of danegaptide completely protected against this increase in permeability (n = 6-8) caused by these RPE barrier stressors (**Figures 1A and 1B,** right column, "+,+").

**Figure 2: Danegaptide protects against DR and AMD-mimicking insult mediated via an improved tight junction organization rate (TiJOR) and improved cell-cell coupling between human retinal pigment epithelial cells in monolayers subjected to stress.**

Human retinal epithelial cells (ARPE-19) were seeded and cultured in microscope chamber slides and grown to confluency to form tight monolayers as visualised by RPE cell-cell couplings in either vehicle (complete media) or danegaptide (DGP, 100 nM) treatment conditions for 24 h. Subsequently, media was replaced with: (i) vehicle (complete media; **Figure 2A,** left column "-,-"; **Figure 2B,** left image); (ii) media supplemented with 30 mM glucose (a hyperglycemia stress) and a sublethal concentration of *tert-butyl* hydroperoxide (*t*BHP, 200 μM) (an oxidative stress) **(Figure 2A,** middle column "+,-"; **Figure 2B,** middle image); or (iii) media supplemented with 30 mM glucose, a sublethal concentration of *tert-butyl* hydroperoxide (tBHP, 200 μM) plus 100 nM Danegaptide **(Figure 2A,** right column "+,+"; **Figure 2B,** right image) and incubated for a period of 48 h. The results are shown in the top graph of **Figure 2 (Figure 2A).**

Tight junction organization was assessed by immunocytochemistry using anti-zonula occludens 1 (ZO-1) immunostaining (bottom images of **Figure 2;** that is, **Figure 2B**), a major component of Tight Junctions that binds the cytoplasmic domain of both the Gap Junction transmembrane protein, Cx43, and the TJ transmembrane proteins, occludin(s) and claudin(s). The Tight Junction organization index (TiJOR) was calculated according to Terryn *et al.,* 2013 and measures the degree of TJ dependent cell-cell coupling. The DR and AMD-mimicking insult of hyperglycemia and oxidative stress significantly decreased TiJOR (n = 13-16). Treatment with 100 nM of danegaptide completely protected against this loss of Tight Junction organization (n = 13-16) and RPE cell-cell uncoupling, which correlates with the ability of danegaptide to protect against RPE barrier dysfunction and pathological RPE paracellular permeability caused by the combination of these hyperglycemic and oxidative cell stressors.

High glucose and oxidative stress (i.e., cellular stress) leads to tight junction disorganization (i.e., reduced TiJOR) and breakdown in cell-cell coupling. These results show that Danegaptide can protect from this uncoupling of cells under stressed conditions. That is, danegaptide can improve TiJOR and maintain cell-cell coupling between RPE cells subjected to stress.

**Figure 3: Blood Glucose Levels (mmol/l) and Blood Glycated Hemoglobin (HbA1C) levels (mmol/mol) during the *in vivo* studies.**

Blood glucose levels were measured by rapid meter every other week after DR induction with STZ in all animals. In those cases, where readings on the rapid meter were out of range, additional plasma samples were collected and measured using a fluorophotometric method. Blood glucose levels were significantly increased by day four after induction compared to the Naive group (Two-Way ANOVA, p < 0.001) (**Figure 3A**).

The levels of glycated hemoglobin (HbA1c) were significantly increased on study day 35, and remained at similar levels at the end of the study (day 60) (Two-Way ANOVA, $p < 0.001$) (**Figure 3B**).

**Figure 4: Cataract scores during the *in vivo* studies.**

The appearance of hyperglycemia-induced cataracts is a common phenotype of the STZ model and was scored on the days of test article administration and at the end of the study period. Cataract appearance was scored from 0, normal lens, to 3, when opacities covered more than 75% of the lens. Naïve animals did not have any cataracts or opacities at any point during the study. The various STZ-induced groups showed a similar degree of cataracts throughout the study after IVT administration, with mild cataracts appearing at day 42 that become more severe reaching moderate severity by day 60 (**Figure 4**). However, there was no significant difference in cataracts between the STZ treatment groups.

**Figure 5: Danegaptide protects against outer retinal thickening in rats with DR after STZ-induction.**

An increase in outer retinal thickness is typically observed during the early phase of STZ-induced DR in the Brown Norway rat. Despite the small absolute increase, which is typically in the range of 3 - 5%, state-of-the-art *in vivo* imaging using SD-OCT can reveal these biologically relevant differences that are indicative of edema or cell swelling indicative of a compromised outer blood retinal barrier.

SD-OCT scans were taken at week nine prior to sampling. The retinal scans were segmented, and the thickness was analyzed by a convolutional neural network algorithm.

Outer retinal thickness was quantified from SD-OCT images from 24 locations to generate a mean thickness of outer retinal thickness. Outliers were removed using the GROUT method (Q = 1%), leading to the removal of a total of 5 outliers (naïve: 0, STZ + Vehicle: 1; STZ + AF564: 1; STZ + Danegaptide: 1; STZ + AF564 + Danegaptide: 2). When tested for normality, SD-OCT data showed a normal distribution.

Streptozotocin (STZ) induction in Brown Norway rats resulted in hyperglycemia, DR and a statistically significant increase in outer retinal thickness in STZ + Vehicle (123.8 ± 0.9 μm; n = 21; t-test $p < 0.01$) retinas after 9 weeks of induction when compared to naïve animals that did not have receive STZ-induction and have DR (119.8 ± 0.5 μm; n = 16; t-test $p < 0.01$), as measured by SD-OCT imaging and quantitation of changes in the outer plexiform, outer nuclear layers, inner and outer segments and RPE/choroid layers with a neural network algorithm **(Figure 5A**).

Danegaptide treatment by localized intravitreal (IVT) eye injections targeting 100 nM of danegaptide in eye, every week for three weeks of treatment prevented this increase in outer retinal thickness (119.7 ± 0.8 μm; n = 27; $p < 0.05$) as measured by SD-OCT imaging, while treatment with an anti-VEGF compound (AF564; 122.7 ± 1.2 μm; n = 25; $p = 0.60$) and anti-VEGF + danegaptide injection treatment (122.3 ± 1.1 μm; n = 17; $p = 0.60$) had no protective effect (**Figure 5B**). Pathological thickening of the oBRB is caused by RPE barrier dysfunction, and this is a hallmark of DME and AMD. The ability of danegaptide to prevent this thickening of the outer retinal layer along in these diabetic rats with DR shows that danegaptide could be an effective therapy for AMD as well as DR and DME (as previously shown).

Representative SD-OCT scans of outer retinal thickness from the left and right eye nine weeks after STZ-induction are shown in **Figure 5C.**

Without wishing to be bound by theory, the inventors believe the increase in outer retinal thickness caused by STZ-induction shows cell swelling as a consequence of cellular stress. It is thought that this is due to Cx43 hemichannel opening, which would allow water to penetrate into the cells. Danegaptide is likely to close the Cx43 hemichannels, thus protecting the subject from cell swelling and thereby outer retinal layer thickening.

**Figure 7: Danegaptide protects against outer retinal thickening throughout the retina.**

Outer retinal thickness was analyzed for different regions of the retina, specifically the superior-nasal (SN), superior-temporal (ST), inferior-nasal (IN), and inferior-temporal (IT) (see **Figure 6** for locations), with 8 locations contributing to each measurement (**Figure 7** and **Table 1**).

**Table 1**. Average value (in μm) of the outer retinal thickness per each treatment group and quadrant

| | ST | SN | IT | IN |
|---|---|---|---|---|
| **Naïve** | 123.99 | 120.83 | 121.62 | 118.56 |
| **STZ + Vehicle** | 126.95 | 125.40 | 127.44 | 126.09 |
| **STZ + AF564** | 123.85 | 123.49 | 126.55 | 123.62 |
| **STZ + Danegaptide** | 118.14 | 117.36 | 123.61 | 121.17 |
| **STZ + AF564 + Danegaptide** | 124.33 | 123.45 | 126.39 | 123.25 |

Outer retinal thickness was statistically significantly increased in STZ + Vehicle-treated rats compared with naïve rats in all regions (**Figures 7A-D;** see also **Table 1** "STZ + Vehicle" as compared to "Naïve"). Danegaptide prevented thickening of the outer retina in all areas (**Figures 7E-H;** see also **Table 1** "STZ + Danegaptide" as compared to "STZ + Vehicle").

**Figure 8. Regional analysis of inner (and total) retinal thickness.**

SD-OCT scans were taken at week nine prior to sampling. Retinal scans were segmented and the thickness was analyzed by a convolutional neural network algorithm. Inner retinal thickness was analyzed by region (**Figure 8**). Regions analyzed were superior-nasal (SN), superior-temporal (ST), inferior-nasal (IN), and inferior-temporal (IT) (see **Figure 6** for

locations).

Retina was significantly thinner in STZ-induced eyes compared to the naïve animals (**Figures 8A-D**). However, no statistically significant differences were identified between the treatment groups (**Figures 8E-H**).

Analysis of total retinal thickness in STZ animals is confounded by decreases in inner retinal thickness and concomitant increases in outer retinal thickness, and therefore, typically do not provide sufficient granularity to dissect pharmacological effects of treatments. Total retinal thickness measurements are summarized in **Table 2.**

**Table 2.** Total retinal thickness (in μm) by region quantified from SD-OCT images

| | **Whole Retina** | **ST** | **SN** | **IT** | **IN** |
|---|---|---|---|---|---|
| **Naïve** | 215 ± 0.6 | 220 ± 0.7 | 214 ± 0.7 | 217 ± 0.7 | 209 ± 0.8 |
| **STZ + Vehicle** | 215 ± 1.8 | 218 ± 3.6 | 211 ± 3.5 | 221 ± 1.8 | 210 ± 2.3 |
| **STZ + AF564** | 213 ± 2.0 | 218 ± 2.6 | 211 ± 2.6 | 219 ± 2.0 | 210 ± 2.6 |
| **STZ + Danegaptide** | 212 ± 2.2 | 210 ± 2.6 | 205 ± 1.9 | 217 ± 1.6 | 206 ± 1.8 |
| **STZ + AF564 + Danegaptide** | 207 ± 2.3 | 218 ± 1.9 | 210 ± 2.0 | 220 ± 1.4 | 208 ± 2.3 |

**Figure 9: Danegaptide and anti-VEGF treatment reduce Evans Blue (EB) extravasation in the diabetic retina.**

Extravasation of Evans Blue is a well-recognized measure to quantify vascular leakage in multiple organ systems. In the case of the retina, extravasation of Evans Blue is detectable in tissue lysates when either (or both) the inner or outer retinal blood barrier are compromised.

To determine the effect of Danegaptide alone or in combination with an anti-VEGF antibody, Evans Blue was injected intravenously and allowed to perfuse for 2 h. After this period, blood samples were collected to determine the amount of Evans Blue in the plasma, which served as normalization factor for quantification of Evans Blue extravasation in the retina. Values presented are the normalized amount of Evans Blue, derived as the concentration of Evans Blue (ng/ml) per milligram tissue weight, divided by the concentration of Evans Blue in the plasma.

One datapoint (in the "STZ + Danegaptide" group) from the entire dataset was excluded based on outlier analysis using the GROUT method with a Q value of 1%. The single outlier was considered a technical artefact as this single value deviated by greater than 30-fold from the measurements of two additional technical replicates of the same sample. Data were subsequently analyzed for normality using the Kolmogorov-Smirnov test. The majority of experimental groups did not follow a normal distribution (**Table 3**), and therefore, the data were log-transformed.

**Table 3**. *P* values for Kolmogorov-Smirnov test for normal and lognormal distributions

| | **Naïve** | **STZ + Vehicle** | **STZ + AF564** | **STZ + Danegaptide** | **STZ + AF564 + Danegaptide** |
|---|---|---|---|---|---|
| *P* value normal distribution | > 0.10 | 0.02 | 0.05 | > 0.10 | 0.02 |
| *P* value lognormal distribution | > 0.10 | > 0.10 | > 0.10 | > 0.10 | > 0.10 |

Given the variability of EB extravasation, which is typical for the STZ model in Brown Norway rats, the effect size of the various treatment groups was analyzed. First, to determine the fold-increase of EB extravasation following STZ, data were normalized to the Naïve group (**Figure 9A**). Induction of STZ resulted in a greater than five-fold increase in EB extravasation 9 weeks after induction (100 ± 13.5% in Naïve vs. 628 ± 212.5% in STZ + Vehicle, n = 10-11, t-test $p < 0.01$).

Subsequently, data were normalized to the STZ + Vehicle group to determine the relative effect size of the IVT treatment interventions. Data were assessed for normality using the D'Agostino & Pearson test for relative data sets, and all STZ groups passed normality.

Anti-VEGF treatment with AF564 resulted in a statistically significant reduction of EB extravasation (17.8 ± 4.3% of STZ + Vehicle, n = 10, One-Way ANOVA $p < 0.01$, Holm-Šídák's multiple comparisons test $p < 0.01$), corresponding to an 82.3% reduction (**Figure 9B**, "STZ + AF564" as compared to "STZ + Vehicle").

Similarly, Danegaptide showed a statistically significant reduction of EB extravasation (31.1 ± 5.3% of STZ + Vehicle, n = 9, One-Way ANOVA $p < 0.01$, Holm-Šídák's multiple comparisons test $p < 0.05$), corresponding to a 68.9% reduction (**Figure 9B,** "STZ + Danegaptide" as compared to "STZ + Vehicle").

The effect of the combination treatment of anti-VEGF and Danegaptide showed a similar effect as Danegaptide alone (34.4. ± 10.3%, n = 10, $p < 0.05$), equivalent to a 65.6% reduction (**Figure 9B**, "STZ + AF564 + Danegaptide" as compared to "STZ + Vehicle").

**Figures 10 & 11: Danegaptide prevents pericyte loss 9 weeks after induction of STZ.**

Vascular abnormalities and the effect of induction of DR by STZ induction were also assessed by quantification of acellular capillaries, pericytes and pericyte ghosts on trypsin-digested retinal wholemounts. For quantification, 4-15 individual images were quantified for each animal and the data from each image were included in the analysis.

Based on the inventors' prior studies performed in the STZ-induced DR model in Brown Norway rats, the most reliable readout to determine vascular abnormalities is the quantification of pericyte ghosts, which appear early in disease and correspond to retinal pericyte loss, whereas acellular capillaries are typically observed at later time points. Specifically, in a pilot study it had been determined that induction of DR by STZ results in a statistically significant increase in pericyte ghosts after 12 weeks. As such, we anticipated to detect the presence of pericyte ghosts, with normal pericyte counts at week 9.

These observations were confirmed in this study as no differences in the total number of pericytes (sum of viable pericytes and pericyte ghosts) or acellular capillaries were observed in STZ-induced animals (**Table 4**; "STZ + Vehicle" as compared to "Naïve"). Furthermore, no significant differences between the various treatment groups were identified (**Table 4**).

**Table 4.** Quantification of pericytes and cellular capillaries

| | **Naïve** | **STZ + Vehicle** | **STZ + AF564** | **STZ + Danegaptide** | **STZ + AF564 + Danegaptide** |
|---|---|---|---|---|---|
| Pericytes* | 52 ± 2 | 52 ± 2 | 47 ± 3 | 49 ± 2 | 51 ± 2 |
| Accellular capillaries | 22 ± 1 | 22 ± 1 | 18 ± 2 | 20 ± 1 | 25 ± 1 |
| (*) sum of the number of pericytes and pericyte ghosts | | | | | |

There was no statistically significant difference in the total number of pericytes, quantified as the sum of the number of pericytes and pericyte ghosts (One-Way ANOVA, $p = 0.51$). Similarly, there were no significant differences in the number of acellular capillaries, except a statistical trend toward fewer acellular capillaries in the anti-VEGF-treated group (STZ + AF564; $p = 0.06$) compared with vehicle-treated (STZ + Vehicle) eyes (One-Way ANOVA, $p < 0.001$ followed by Holm-Šídák'smultiple comparisons test).

Subsequently, number of pericyte ghosts was quantified. Outliers were removed from the data set using the GROUT method (Q = 1%), which resulted in the removal 19 of the total of 324 data points collected.

To validate the model based on the presence of pericyte ghosts, the number of pericyte ghosts in the retina of the naïve group was first compared to the number in the retina of vehicle-treated STZ-induced animals. STZ induction resulted in a statistically significant increase in the number of pericyte ghosts in the retina compared with naïve rats (**Figure 10A**).

Subsequently, STZ groups were compared by One-Way ANOVA ($p < 0.05$) followed by Dunnett's multiple comparisons test (**Figure 10B**). Treatment with both the anti-VEGF antibody (AF564; $p < 0.01$) and Danegaptide ($p < 0.001$) resulted in a statistically significant reduction of pericyte ghosts, while the combination treatment did not show a statistical effect ($p = 0.11$).

Representative examples of trypsin digested, PAS-stained retinal wholemounts are shown in **Figure 11,** which shows acellular capillaries (arrows), pericytes (not indicated) and pericytes ghost (arrowheads).

**Conclusions**

Exposure of ARPE-19 cells (48 h) to sublethal oxidative stress and hyperglycemia resulted in a statistically significant increase in $P_{app}$ (**Figures 1A and 1B**, middle column, "+,-"; as compared to the control left column, "-,-"), and a concomitant decrease in the TiJOR (**Figure 2A**, middle column, "+,-"; as compared to the control left column, "-,-"). Pre-treatment with danegaptide (100 nM) fully protected against this increase in permeability (**Figures 1A and 1B**, right column, "+,+"; as compared to the control left column, "-,-" and middle column, "+,-"). Similarly, danegaptide showed partial protection against tight junction disorganization and breakdown in cell-cell coupling as assessed by quantification of TiJOR (**Figure 2A**, right column, "+,+"; as compared to the control left column, "-,-" and middle column, "+,-").

STZ resulted in reliable induction of hyperglycemia, as quantified by sustained elevated glucose (**Figure 3A**) and hemoglobin A1c levels (**Figure 3B**) throughout the study. STZ-induced cataracts are common in this model and developed moderate severity by the end of the study (**Figure 4**). All test articles were well-tolerated, and no signs of ocular toxicity were identified by macroscopic ophthalmic examinations. Test articles exerted no differential effects on cataract development (**Figure 4**).

Induction of DR by STZ resulted in a statistically significant increase in outer retinal thickness (**Figure 5B**; "STZ + Vehicle" as compared to "Naïve") that was fully reversed by Danegaptide ($p < 0.05$) (**Figure 5B**; "STZ + Danegaptide"). In

contrast, AF564 ($p$ = 0.60) (**Figure 5B**; "STZ + AF564") and the combination treatment ($p$ = 0.60) (**Figure 5B**; "STZ + AF564 + Danegaptide") had no effect. This reduction in outer retinal thickness by Danegaptide was shown throughout the retina (**Figure 7**).

The positive control, anti-VEGF antibody AF564, resulted in a statistically significant 82.3% reduction of EB extravasation ($p < 0.01$) (**Figure 9B**; "STZ + AF564" as compared to "STZ + Vehicle"). Similarly, Danegaptide resulted in a 68.9% reduction of EB extravasation ($p < 0.05$) (**Figure 9B**; "STZ + Danegaptide" as compared to "STZ + Vehicle"). The combination treatment of AF564 and Danegaptide showed a similar effect in EB extravasation as Danegaptide alone (65.6% reduction, $p < 0.05$) (**Figure 9B**; "STZ + AF564 + Danegaptide" as compared to "STZ + Vehicle" and "STZ + Danegaptide").

The number of pericyte ghosts was significantly increased in STZ rats (p < 0.05) (**Figure 10A**; "STZ + Vehicle" as compared to "Naïve"). Both AF564 (p < 0.01) (**Figure 10B**; "STZ + AF564") and Danegaptide ($p < 0.001$) (**Figure 10B**; "STZ + Danegaptide") elicited a statistically significant reduction of pericyte ghosts, while the combination treatment had no effect ($p$ = 0.11) (**Figure 10B**; "STZ + AF564 + Danegaptide").

No biologically relevant effects of Danegaptide was identified on inner retinal thickness.

These combined findings (**Figures 1-11**) that danegaptide can protect from RPE barrier dysfunction as shown by danegaptide's ability to prevent increases in RPE monolayer permeability due to hyperglycemic and oxidative stress in a TJ associated manner, and its ability to prevent outer retinal layer thickening in rats with DR indicate the danegaptide could be an effective treatment for patients with d-AMD, wet AMD or neovascular AMD.

Danegaptide protects from loss of blood retinal barrier (BRB) integrity, in part, via Connexin 43 (Cx43) in Gap Junctions by:

- Inner BRB: Protects from loss of pericytes and endothelial cells and vascular leakage as observed not only in DR but also to recently recognized in patients with AMD (see, for example, Hudson *et al.,* 2020 and Hadziahmetovic *et al.,* 2021); and
- Outer BRB: Protects from loss of retinal pigmented epithelial cells coupling barrier integrity leading to reduced barrier integrity and associated functions of the RPE cell layer, including active transport water out of the subretinal space and vascular leakage causing wet AMD.

Accordingly, danegaptide exerts potent protection against experimental conditions that mimic known pathologies of diabetic retinopathy (DR)- and age-related macular degeneration (AM D)-associated with breakdown of tight junctions and cell-cell coupling. Additionally, danegaptide by protecting from pathological Cx43 based hemichannel opening can protect from cell swelling, outer retinal cell layer thickening and pathological leakage of ATP into the extracellular compartment, where ATP in known to cause inflammation. Danegaptide was further well tolerated in rats and resulted in significant protection against stress and DR-associated pericyte loss, retinal vascular leakage and break down of the blood-retinal barriers (both inner and outer BRBs). The level of protection elicited by danegaptide on vascular leakage and pericyte loss was similar to that observed by anti-VEGF treatment. In addition, danegaptide protected against stress and DR-induced increases in outer retinal thickness, which was not shown with anti-VEGF treatment.

## References

Aonuma et al., Chem. Pharm. Bull. (Tokyo), 1980, 28, 3332-3339.
Bandello *et al.,* F1000Research, **2017,** 6, 245.
Butera et al., J. Med. Chem., 2009, 52(4), 908-911.
Clapp et al., Acta Diabetologica, 2019, 56, 1031-1036.
Coutinho et al., Drug Deliv. Transl. Res., 2020, 10(3), 751-765.
Dhein et al., Naunyn Schmiedebergs Arch Pharmacol., 1994, 350, 174-184.
Ferris et al., Ophthalmology, 2013, 120(4), 844-851.
Fields et al., "Principles and practice of solid-phase peptide synthesis", Synthetic Peptides (2002, 2nd Edition)
Filek et al., Clin. Ophthalmol., 2019, 13, 1087-96.
González-Casanova et al., Int. J. Mol. Sci., 2021, 22(6), 3194.
Guo et al., 2016, 57(10), 3961-3973.
Hadziahmetovic et al., Front. Cell Dev. Biol., 2021, 8, 612812.
Hudson et al., Neural Regen. Res., 2020, 15(9), 1656-1657.
King et al., Int. J. Mol. Sci., 2021, 22, 10186.
Mat et al., 2019, 17(1), 371-387.

Mugisho et al., Biochimica et Biophysica Acta (BBA) - General Subjects, 2019, 1862 3, 385-393.
Muller et al., Eur. J. Pharmacol., 1997, 327, 65-72.
Naylor et al., Int. J. Mol. Sci., 2020, 21(1), 211.
Obert et al., J. Mol. Med. (Berl), 2017, 95(5), 535-552.
Song et al., Neuro-Oncology, 2005, 7(4), 453-464.
Squires et al., Int. J. Mol. Sci., 2021, 22(6), 2809.
Terryn et al., Cytometry A, 2013; 83, 235-241.
U.S. Patent No. 4,775,743
WO 02/077017
WO 2007/078990
WO 2008/079266
WO 2018/202865
Ziniauskaité et al., Ocul Surf., 2019, 17, 257-264.

## Claims

**1.** A compound of formula (I):

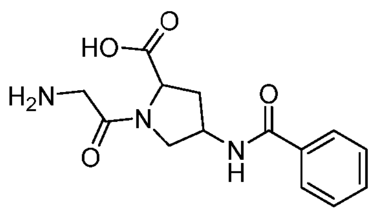
(I),

or a pharmaceutically acceptable salt or hydrate thereof,
for use in a method for treating or preventing age-related macular degeneration (AMD) in a human subject, the method comprising administering to the subject a therapeutically effective amount of the compound, or a pharmaceutically acceptable salt or hydrate thereof.

**2.** The compound for use according to claim 1, wherein the compound is (2S,4R)-1-(2-aminoacetyl)-4-benzoylamino-pyrrolidine-2-carboxylic acid, or a pharmaceutically acceptable salt or hydrate thereof.

**3.** The compound for use according to claim 1 or 2, wherein the compound or a pharmaceutically acceptable salt or hydrate thereof is administered locally to the eye;
optionally wherein the compound or a pharmaceutically acceptable salt or hydrate thereof is administered locally via intravitreal injection in the eye.

**4.** The compound for use according to claim 1 or 2, wherein the compound or a pharmaceutically acceptable salt or hydrate thereof is administered systemically;
optionally wherein the compound or a pharmaceutically acceptable salt or hydrate thereof is administered systemically via oral, subcutaneous, transdermal, or intravenous administration; optionally wherein the compound or a pharmaceutically acceptable salt or hydrate thereof is administered systemically via oral administration.

**5.** The compound for use according to any one of claims 1-4, wherein the method prevents AMD in a human subject and/or prevents the progression of AMD in a human subject;
optionally wherein the method prevents the progression of dry AMD to wet AMD in a human subject, or wherein the method prevents the progression of intermediate dry AMD to advanced forms of dry AMD in a human subject.

**6.** The compound for use according to any one of claims 1-5, wherein the AMD is characterized as:

(a) early stage dry AMD;
(b) intermediate stage dry AMD;
(c) advanced stage dry AMD, optionally wherein the advanced stage dry AMD is geographic atrophy; or
(d) wet, neovascular, or advanced angiogenic AMD.

**7.** The compound for use according to any one of claims 1-6, wherein the method further prevents the development of choroidal neovascularization in the human subject.

**8.** The compound for use according to any one of claims 1-7, wherein the subject further:

(a) has chronically high blood glucose levels including for a subject that is brought into glycaemic control;
(b) has type 1 diabetes or type 2 diabetes;
(c) has hypertension or chronically high blood pressure including for a subject that is brought into normal blood pressure control, optionally wherein the subject is brought into normal blood pressure control by anti-hypertensive treatment, glycaemic control, or reductions in cholesterol;
(d) has high cholesterol;
(e) has diabetic retinopathy or diabetic macular edema;
(f) has retinal vein occlusion (RVO) eye disease;
(g) has glaucoma disease with or without pathological intraocular pressure;
(h) has uveitis or other forms of inflammatory eye disease; and/or
(i) has or had drusen deposits or protein exudates in the eye.

**9.** The compound for use according to any one of claims 1-8, wherein the compound:

(a) inhibits dysfunction of the RPE (retinal pigmented epithelium) or the outer blood retina barrier (oBRB); optionally wherein the compound inhibits loss of barrier integrity between the RPE cells and loss of RPE cells;
(b) inhibits stress induced dysfunction or the retinal or choroidal layers in the eye;
(c) inhibits the loss of photoreceptors in the eye; optionally wherein the compound inhibits the loss of photoreceptors in the retina;
(d) wherein the compound inhibits vascular leakage in the retina; optionally wherein the compound inhibits vascular leakage that leads to macular edema;
(e) improves fluid extraction from the retinal tissues via the retinal pigmented epithelium;
(f) inhibits death or loss of retinal endothelial cells, choroid endothelial cells, pericytes or epithelial cells;
(g) inhibits pathological hemichannel opening; and/or inhibits pathological hemichannel ATP release; optionally wherein the pathological hemichannel opening and/or ATP release is Connexin 43 mediated opening and/or ATP release.

**10.** The compound for use according to any one of claims 1-9, wherein the compound or pharmaceutically acceptable salt or hydrate thereof is administered as an oral, systemic, or sustained-release compound formulation.

**11.** The compound for use according to any one of claims 1-10, wherein the concentration of the administered compound that reaches the microenvironment of the eye is about 50 nM to about 150 nM; preferably about 100 nM.

**12.** The compound for use according to any one of claims 1-11, wherein the compound is administered to the subject once or twice daily, or wherein the compound is administered to the subject four times daily.

## Patentansprüche

**1.** Verbindung der Formel (I):

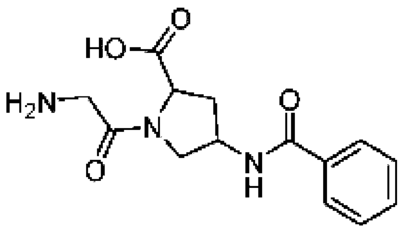

(I)

oder ein pharmazeutisch annehmbares Salz oder Hydrat davon zur Verwendung in einem Verfahren zur Behandlung oder Prävention von altersbedingter Makuladegeneration (AMD) bei einem menschlichen Individuum, wobei das Verfahren das Verabreichen einer therapeutisch wirksamen Menge der Verbindung oder eines pharmazeutisch

annehmbaren Salzes oder Hydrats davon an das Individuum umfasst.

2. Verbindung zur Verwendung nach Anspruch 1, wobei die Verbindung (2S,4R)-1-(2-Aminoacetyl)-4-benzoylaminopyrrolidin-2-carbonsäure oder ein pharmazeutisch annehmbares Salz oder Hydrat davon ist.

3. Verbindung zur Verwendung nach Anspruch 1 oder 2, wobei die Verbindung oder ein pharmazeutisch annehmbares Salz oder Hydrat davon lokal an das Auge verabreicht wird;
wobei die Verbindung oder ein pharmazeutisch annehmbares Salz oder Hydrat davon gegebenenfalls lokal über intravitreale Injektion in das Auge verabreicht wird.

4. Verbindung zur Verwendung nach Anspruch 1 oder 2, wobei die Verbindung oder ein pharmazeutisch annehmbares Salz oder Hydrat davon systemisch verabreicht wird:
wobei die Verbindung oder ein pharmazeutisch annehmbares Salz oder Hydrat davon gegebenenfalls über orale, subkutane, transdermale oder intravenöse Verabreichung systemisch verabreicht wird; wobei die Verbindung oder ein pharmazeutisch annehmbares Salz oder Hydrat davon gegebenenfalls über orale Verabreichung systemisch verabreicht wird.

5. Verbindung zur Verwendung nach einem der Ansprüche 1 bis 4, wobei das Verfahren AMD bei einem menschlichen Individuum verhindert und/oder das Fortschreiten von AMD bei einem menschlichen Individuum verhindert;
wobei das Verfahren gegebenenfalls das Fortschreiten von trockener AMD zu feuchter AMD bei einem menschlichen Individuum verhindert oder wobei das Verfahren das Fortschreiten von intermediären Formen von trockener AMD zu fortgeschrittenen Formen von trockener AMD bei einem menschlichen Individuum verhindert.

6. Verbindung zur Verwendung nach einem der Ansprüche 1 bis 5, wobei die AMD gekennzeichnet ist als:

(a) trockene AMD im frühen Stadium;
(b) trockene AMD im intermediären Stadium;
(c) trockene AMD im fortgeschrittenen Stadium, wobei die trockene AMD im fortgeschrittenen Stadium gegebenenfalls geographische Atrophie ist; oder
(d) feuchte, neovaskuläre oder fortgeschrittene angiogene AMD.

7. Verbindung zur Verwendung nach einem der Ansprüche 1 bis 6, wobei das Verfahren weiters die Entwicklung von choroidaler Neovaskularisation im menschlichen Individuum verhindert.

8. Verbindung zur Verwendung nach einem der Ansprüche 1 bis 7, wobei das Individuum weiters:

(a) einen chronisch erhöhten Blutzuckerspiegel aufweist, einschließlich eines Individuums, bei dem der Blutzucker eingestellt wurde;
(b) Typ-1-Diabetes oder Typ-2-Diabetes aufweist;
(c) Hypertonie oder chronischen Blutdruckhochdruck aufweist, einschließlich eines Individuums, bei dem der Blutdruck auf normal gebracht wurde, wobei bei dem Individuum gegebenenfalls der Blutdruck durch antihypertensive Behandlung, Blutzuckereinstellung oder Cholesterinreduktion auf normal gebracht wurde;
(d) einen hohen Cholesterinspiegel aufweist;
(e) diabetische Retinopathie oder diabetisches Makulaödem aufweist;
(f) eine Retinaler-Venenverschluss- (RVO-) Erkrankung aufweist;
(g) eine Glaukom-Erkrankung mit oder ohne pathologischen intraokulären Druck aufweist;
(h) Uveitis oder andere Formen von entzündlicher Augenerkrankung aufweist;
und/oder

(i) Drusenablagerungen oder Proteinexsudate im Auge aufweist oder aufgewiesen hat.

9. Verbindung zur Verwendung nach einem der Ansprüche 1 bis 8, wobei die Verbindung:

(a) eine Dysfunktion des RPE (retinalen Pigmentepithels) oder der äußeren Blut-Retina-Schranke (oBRB) hemmt; wobei die Verbindung gegebenenfalls einen Verlust der Schrankenunversehrtheit zwischen den RPE-Zellen und einen Verlust von RPE-Zellen hemmt;
(b) eine stressinduzierte Dysfunktion der retinalen oder choroidalen Schichten im Auge hemmt;
(c) einen Verlust von Photorezeptoren im Auge hemmt; wobei die Verbindung gegebenenfalls einen Verlust von

Photorezeptoren in der Retina hemmt;
(d) wobei die Verbindung vaskulären Auslauf in der Retina hemmt; wobei die Verbindung gegebenenfalls zu Makulaödem führenden vaskulären Auslauf hemmt;
(e) die Fluidextraktion aus retinalem Gewebe über das retinale Pigmentepithel verbessert;
(f) ein Absterben oder einen Verlust von retinalen Endothelzellen, choroidalen Endothelzellen, Perizyten oder Epithelzellen hemmt;
(g) eine pathologische Hemikanal-Öffnung hemmt; und/oder eine pathologische Hemikanal-ATP-Freisetzung hemmt; wobei die pathologische Hemikanal-Öffnung und/oder ATP-Freisetzung gegebenenfalls eine Connexin-43-vermittelte Öffnung und/oder ATP-Freisetzung ist.

**10.** Verbindung zur Verwendung nach einem der Ansprüche 1 bis 9, wobei die Verbindung oder ein pharmazeutisch annehmbares Salz oder Hydrat davon als orale oder systemische Verbindungsformulierung oder als Verbindungsformulierung mit verzögerter Freisetzung verabreicht wird.

**11.** Verbindung zur Verwendung nach einem der Ansprüche 1 bis 10, wobei die Konzentration der verabreichten Verbindung, welche die Mikroumgebung des Auges erreicht, etwa 50 nM bis etwa 150 nM, vorzugsweise etwa 100 nM, beträgt.

**12.** Verbindung zur Verwendung nach einem der Ansprüche 1 bis 11, wobei die Verbindung dem Individuum einmal oder zweimal täglich verabreicht wird oder wobei die Verbindung dem Individuum viermal täglich verabreicht wird.

## Revendications

**1.** Composé de formule (I) :

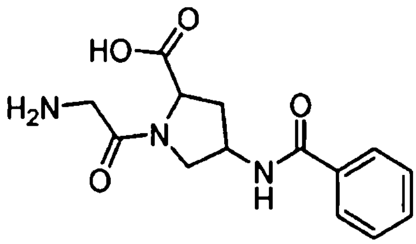

(I),

ou un sel ou un hydrate pharmaceutiquement acceptable de celui-ci,
pour utilisation dans un procédé de traitement ou de prévention de la dégénérescence maculaire liée à l'âge (DMLA) chez un sujet humain, le procédé comprenant une administration au sujet d'une quantité thérapeutiquement efficace du composé, ou d'un sel ou d'un hydrate pharmaceutiquement acceptable de celui-ci.

**2.** Composé pour utilisation selon la revendication 1, dans lequel le composé est l'acide (2S,4R)-1-(2-aminoacétyl)-4-benzoylamino-pyrrolidine-2-carboxylique, ou un sel ou un hydrate pharmaceutiquement acceptable de celui-ci.

**3.** Composé pour utilisation selon la revendication 1 ou 2, dans lequel le composé ou un sel ou un hydrate pharmaceutiquement acceptable de celui-ci est administré localement à l'œil ;
facultativement dans lequel le composé ou un sel ou un hydrate pharmaceutiquement acceptable de celui-ci est administré localement par injection intravitréenne dans l'œil.

**4.** Composé pour utilisation selon la revendication 1 ou 2, dans lequel le composé ou un sel ou un hydrate pharmaceutiquement acceptable de celui-ci est administré de manière systémique ;
facultativement dans lequel le composé ou un sel ou un hydrate pharmaceutiquement acceptable de celui-ci est administré de manière systémique par administration orale, sous-cutanée, transdermique ou intraveineuse ; facultativement dans lequel le composé ou un sel ou un hydrate pharmaceutiquement acceptable de celui-ci est administré de manière systémique par administration orale.

**5.** Composé pour utilisation selon l'une quelconque des revendications 1 à 4, dans lequel le procédé empêche la DMLA chez un sujet humain et/ou empêche la progression de la DMLA chez un sujet humain ;

facultativement dans lequel le procédé empêche la progression de la DMLA sèche en DMLA humide chez un sujet humain, ou dans lequel le procédé empêche la progression de la DMLA sèche intermédiaire en formes avancées de DMLA sèche chez un sujet humain.

**6.** Composé pour utilisation selon l'une quelconque des revendications 1 à 5, dans lequel la DMLA est caractérisée comme :

(a) une DMLA sèche à un stade précoce ;
(b) une DMLA sèche à un stade intermédiaire ;
(c) une DMLA sèche à un stade avancé, facultativement dans lequel la DMLA sèche à un stade avancé est une atrophie géographique ; ou
(d) une DMLA humide, néovasculaire ou angiogénique avancée.

**7.** Composé pour utilisation selon l'une quelconque des revendications 1 à 6, dans lequel le procédé empêche en outre le développement d'une néovascularisation choroïdienne chez le sujet humain.

**8.** Composé pour utilisation selon l'une quelconque des revendications 1 à 7, dans lequel en outre le sujet :

(a) présente des taux de glycémie chroniquement élevés, y compris pour un sujet qui est soumis à un contrôle glycémique ;
(b) est diabétique de type 1 ou de type 2 ;
(c) présente une hypertension ou une pression artérielle chronique, y compris pour un sujet qui est soumis à un contrôle de pression artérielle normale, facultativement dans lequel le sujet est soumis à un contrôle de pression artérielle normale par un traitement anti-hypertenseur, un contrôle glycémique ou des réductions du cholestérol ;
(d) présente un taux de cholestérol élevé ;
(e) présente une rétinopathie diabétique ou un œdème maculaire diabétique ;
(f) présente une maladie occulaire d'occlusion de la veine rétinienne (OVR) ;
(g) présente un glaucome avec ou sans pression intraoculaire pathologique ;
(h) présente une uvéite ou d'autres formes de maladie inflammatoire des yeux ; et/ou
(i) présente ou a présenté des dépôts de drusen ou des exsudats protéiques dans l'œil.

**9.** Composé pour utilisation selon l'une quelconque des revendications 1 à 8, dans lequel le composé :

(a) inhibe le dysfonctionnement de l'EPR (épithélium pigmenté rétinien) ou de la barrière hémato-rétinienne externe (oBRB) ; facultativement, dans lequel le composé inhibe la perte d'intégrité de la barrière entre les cellules d'EPR et la perte de cellules d'EPR ;
(b) inhibe un dysfonctionnement induit par le stress ou les couches rétiniennes ou choroïdiennes dans l'œil ;
(c) inhibe la perte de photorécepteurs dans l'œil ; facultativement dans lequel le composé inhibe la perte de photorécepteurs dans la rétine ;
(d) dans lequel le composé inhibe une fuite vasculaire dans la rétine ; facultativement dans lequel le composé inhibe une fuite vasculaire qui conduit à un œdème maculaire ;
(e) améliore l'extraction de liquide à partir des tissus rétiniens via l'épithélium pigmenté rétinien ;
(f) inhibe la mort ou la perte de cellules endothéliales rétiniennes, de cellules endothéliales choroïdiennes, de péricytes ou de cellules épithéliales ;
(g) inhibe l'ouverture pathologique des canaux hématopoïétiques ; et/ou inhibe la libération pathologique d'ATP par les hémicanaux ; facultativement, l'ouverture pathologique des hémicanaux et/ou la libération d'ATP est une ouverture à médiation par la Connexine 43 et/ou une libération d'ATP.

**10.** Composé pour utilisation selon l'une quelconque des revendications 1 à 9, dans lequel le composé ou un sel ou un hydrate pharmaceutiquement acceptable de celui-ci est administré sous la forme d'une formulation de composé à libération orale, systémique ou prolongée.

**11.** Composé pour utilisation selon l'une quelconque des revendications 1 à 10, dans lequel la concentration du composé administré qui atteint le micro-environnement de l'œil est d'environ 50 nM à environ 150 nM ; de préférence d'environ 100 nM.

**12.** Composé pour utilisation selon l'une quelconque des revendications 1 à 11, dans lequel le composé est administré au sujet une ou deux fois par jour, ou dans lequel le composé est administré au sujet quatre fois par jour.

## *Figure 1*

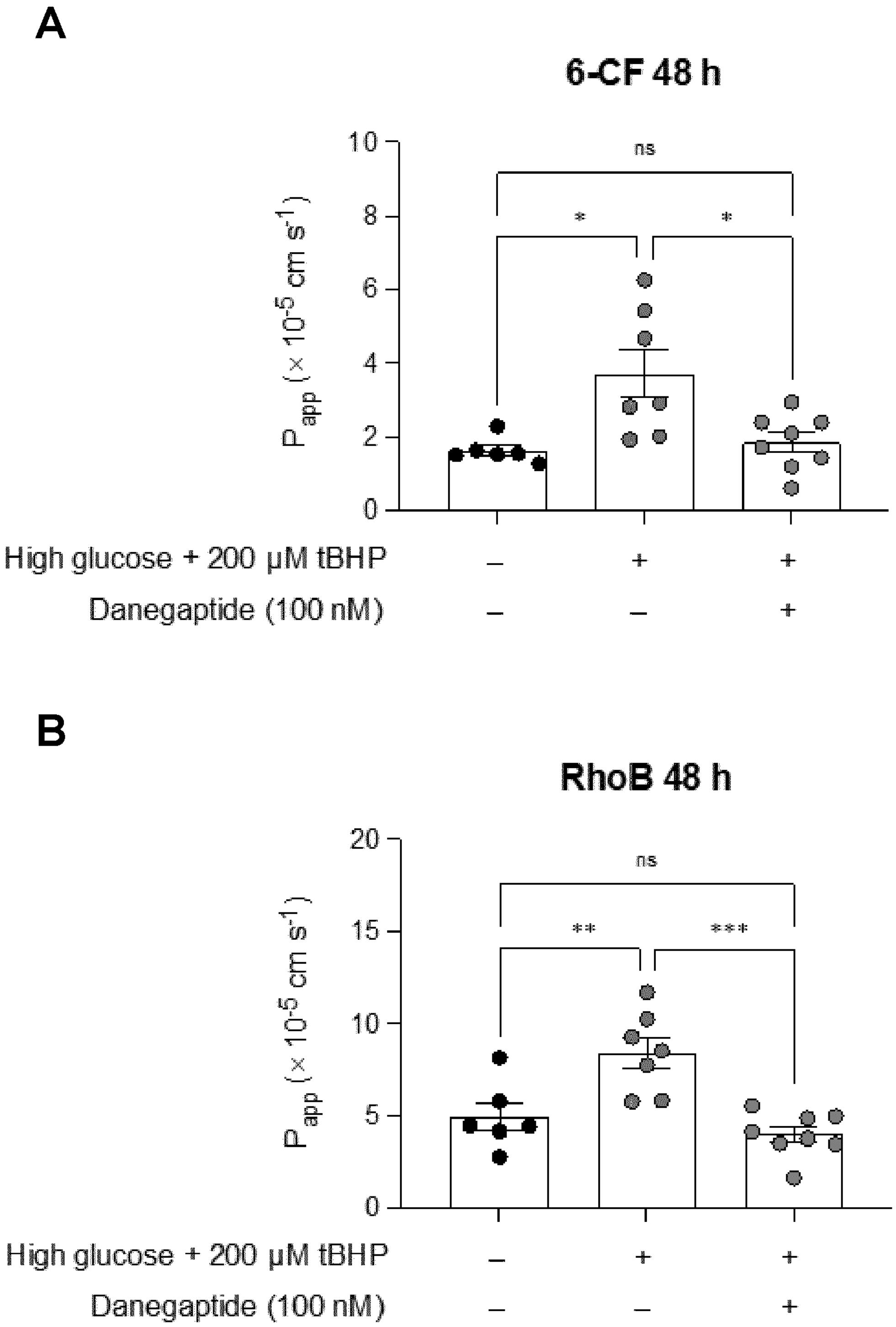
A
6-CF 48 h
$P_{app}$ ($\times 10^{-5}$ cm $s^{-1}$)
0
2
4
6
8
10
ns
*
*
High glucose + 200 µM tBHP – + +
Danegaptide (100 nM) – – +
B
RhoB 48 h
$P_{app}$ ($\times 10^{-5}$ cm $s^{-1}$)
0
5
10
15
20
ns
**
***
High glucose + 200 µM tBHP – + +
Danegaptide (100 nM) – – +

# *Figure 2*

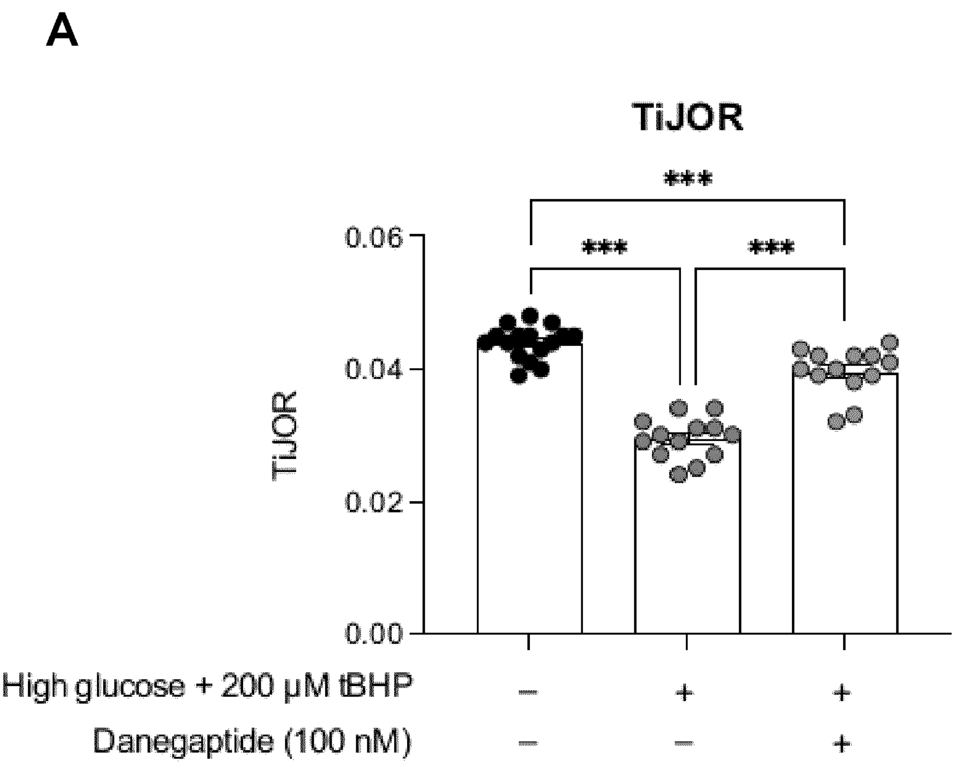
A
TiJOR
***
***
***
0.06
0.04
0.02
0.00
TiJOR
High glucose + 200 µM tBHP
–
+
+
Danegaptide (100 nM)
–
–
+

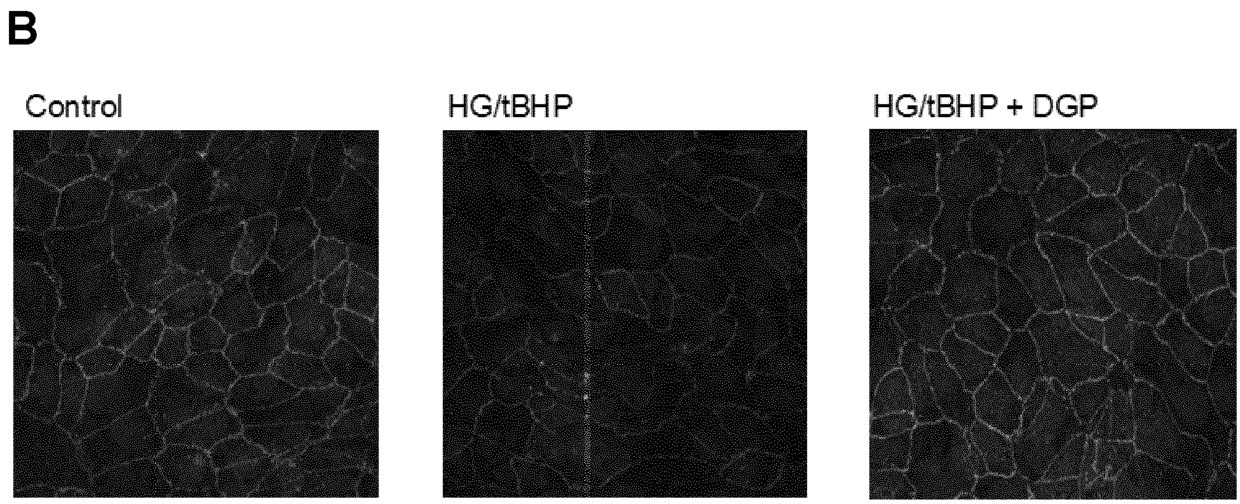
B
Control
HG/tBHP
HG/tBHP + DGP

## *Figure 3*

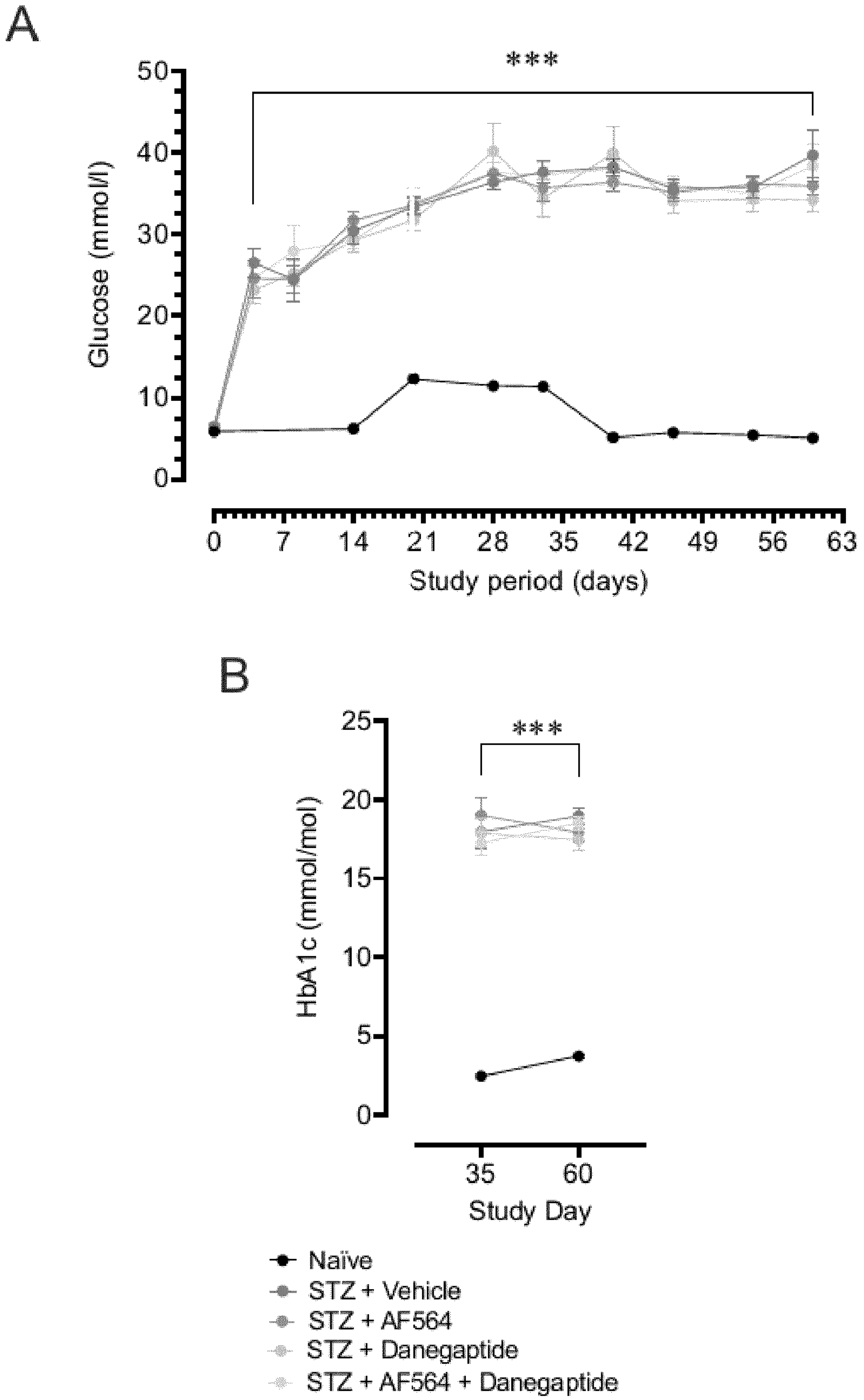
A
***
50
40
30
20
10
0
Glucose (mmol/l)
0
7
14
21
28
35
42
49
56
63
Study period (days)
B
***
25
20
15
10
5
0
HbA1c (mmol/mol)
35
60
Study Day
Naïve
STZ + Vehicle
STZ + AF564
STZ + Danegaptide
STZ + AF564 + Danegaptide

*Figure 4*

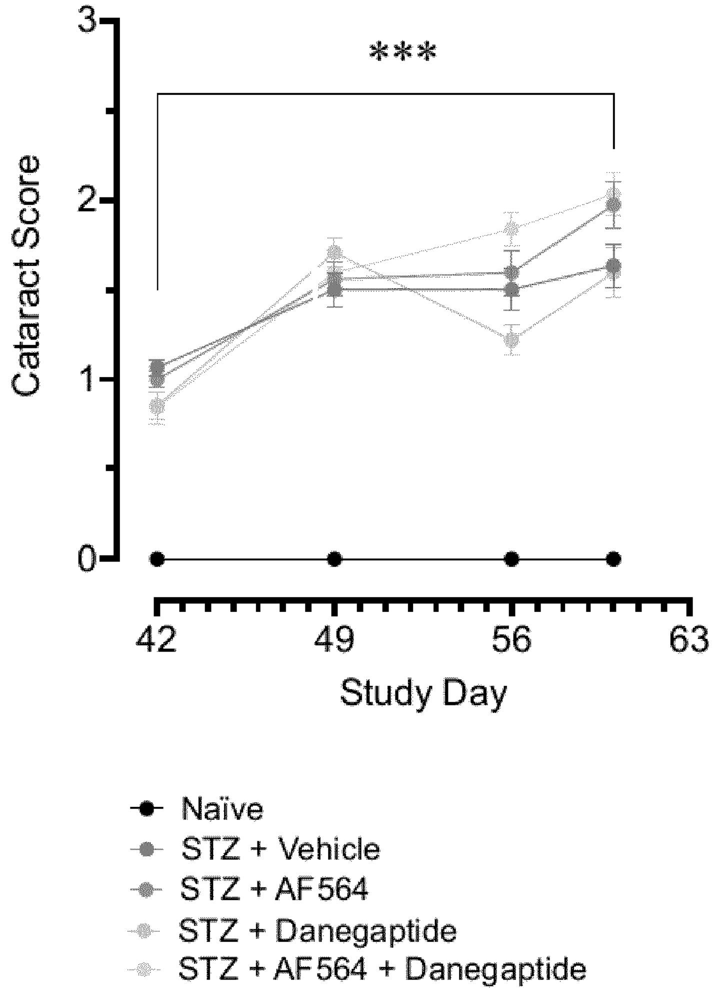
3
2
1
0
***
Cataract Score
42
49
56
63
Study Day
Naïve
STZ + Vehicle
STZ + AF564
STZ + Danegaptide
STZ + AF564 + Danegaptide

***Figure 5***

A

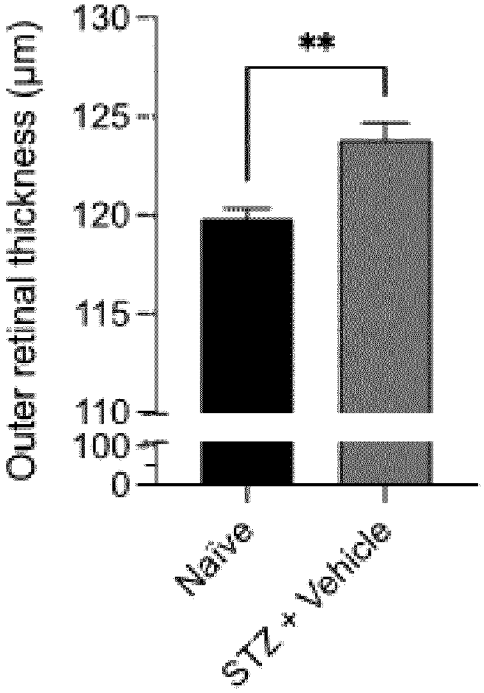
Outer retinal thickness (µm)
130
125
120
115
110
100
0
**
Naive
STZ + Vehicle

B

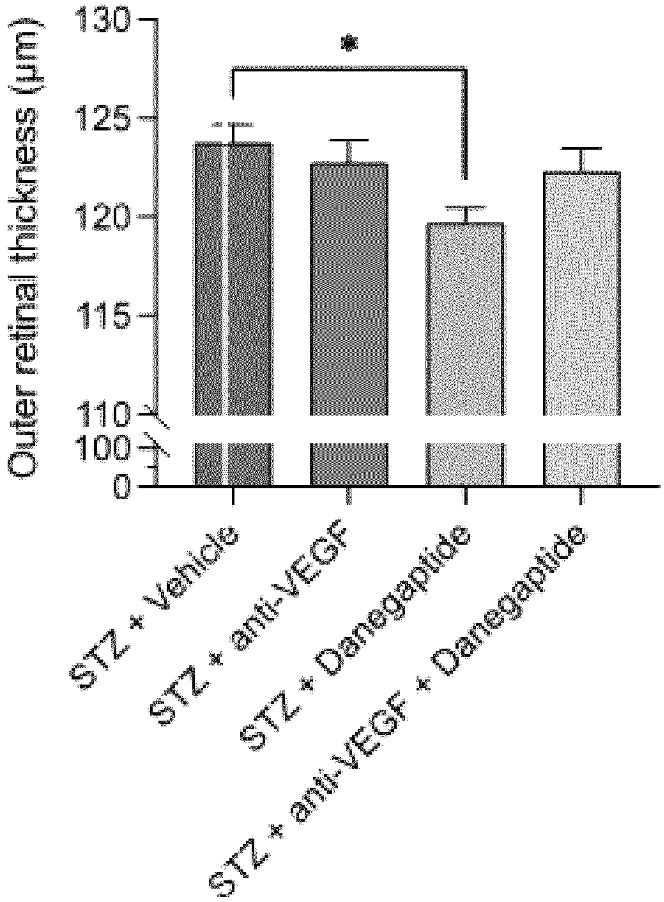
Outer retinal thickness (µm)
130
125
120
115
110
100
0
*
STZ + Vehicle
STZ + anti-VEGF
STZ + Danegaptide
STZ + anti-VEGF + Danegaptide

*Figure 5 (continued)*

C

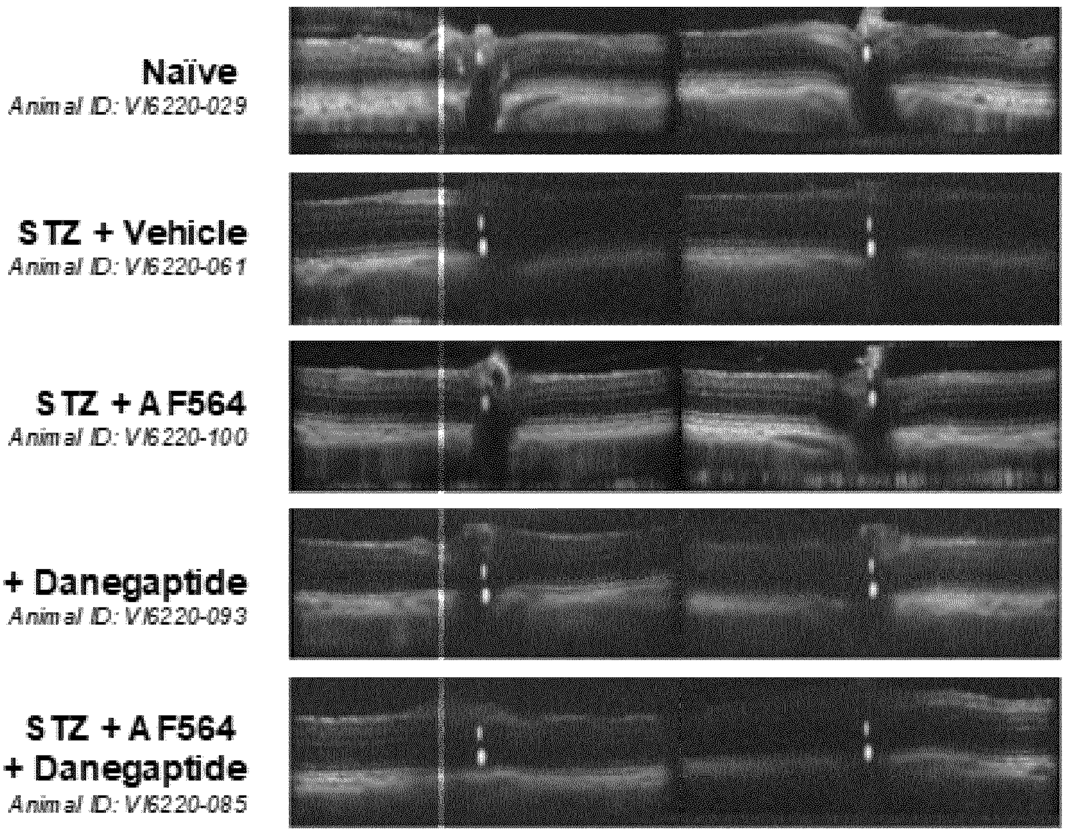

*Figure 6*

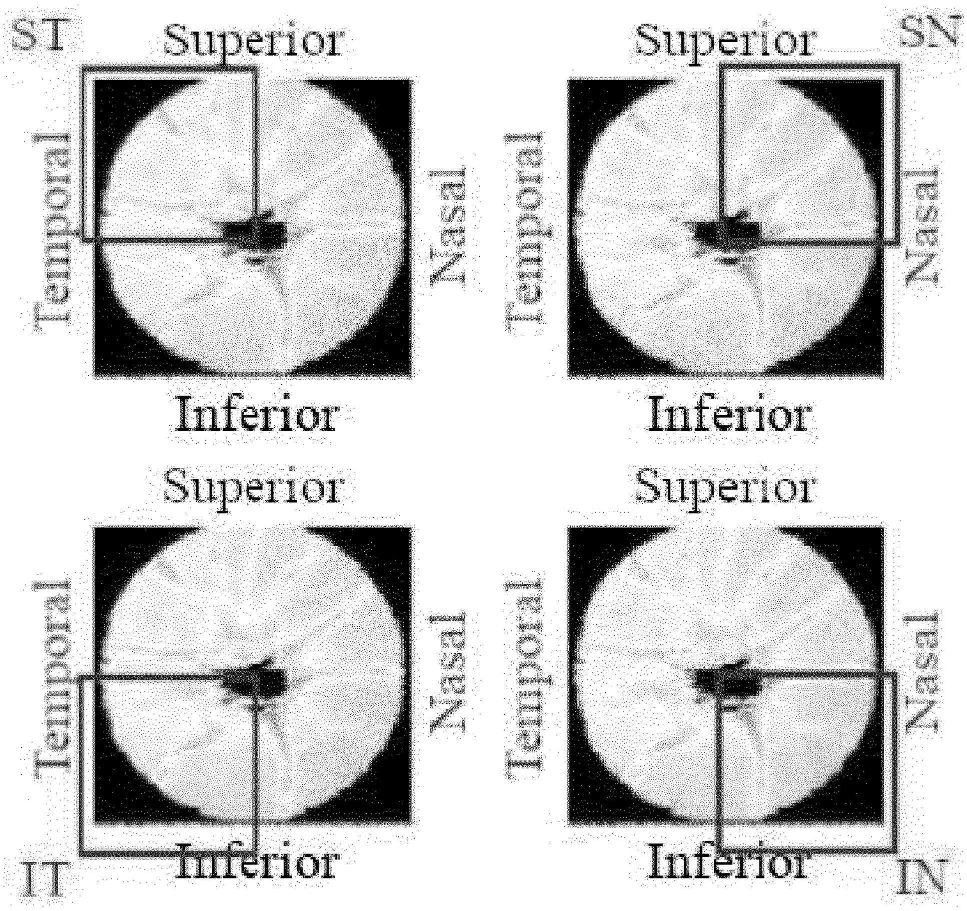
ST
Superior
SN
Superior
Temporal
Nasal
Temporal
Nasal
Inferior
Inferior
Superior
Superior
Temporal
Nasal
Temporal
Nasal
IT
Inferior
Inferior
IN

*Figure 7*

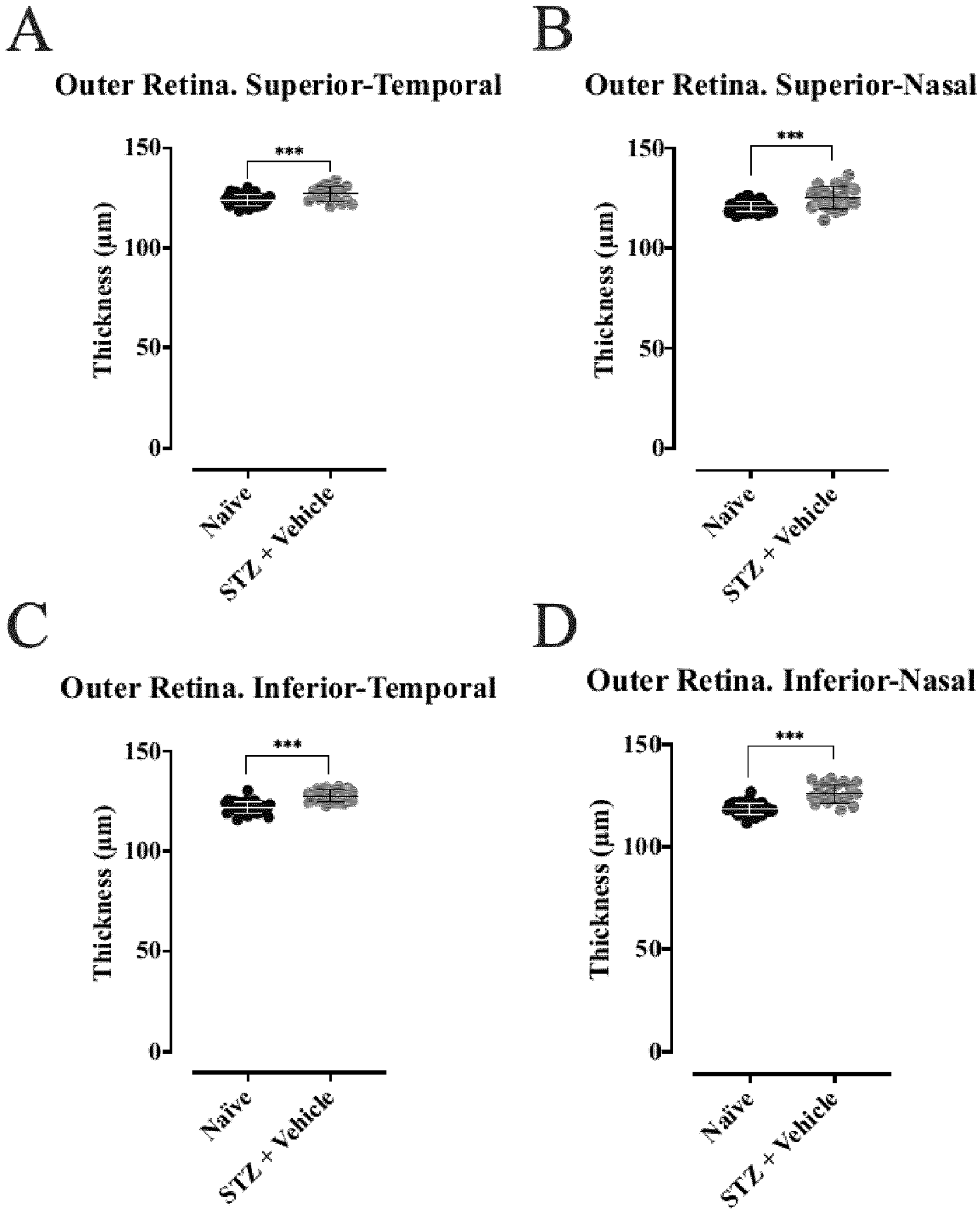
A
Outer Retina. Superior-Temporal
***
Thickness (µm)
150
100
50
0
Naïve
STZ + Vehicle
B
Outer Retina. Superior-Nasal
***
Thickness (µm)
150
100
50
0
Naïve
STZ + Vehicle
C
Outer Retina. Inferior-Temporal
***
Thickness (µm)
150
100
50
0
Naïve
STZ + Vehicle
D
Outer Retina. Inferior-Nasal
***
Thickness (µm)
150
100
50
0
Naïve
STZ + Vehicle

## *Figure 7 (continued)*

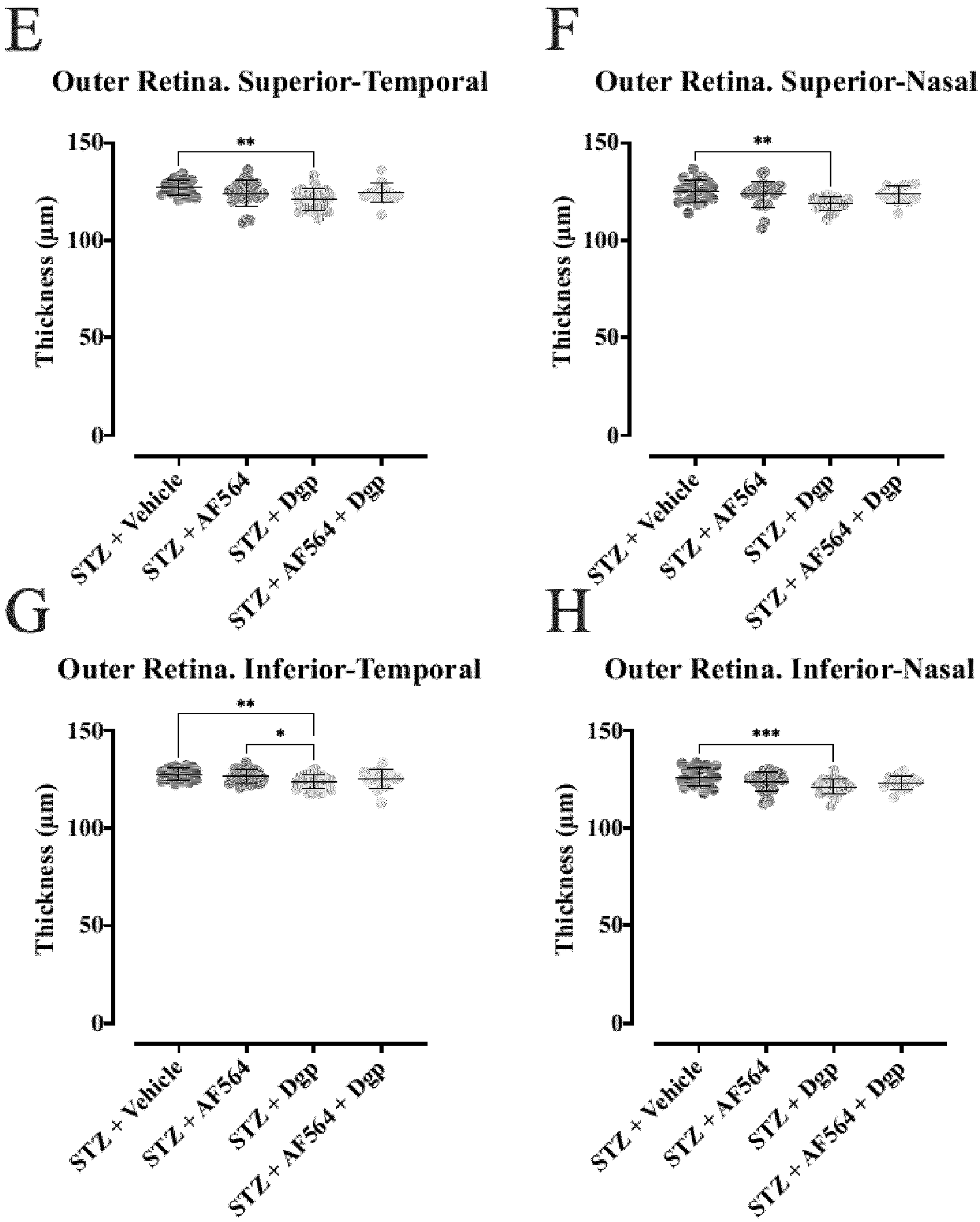

*Figure 8*

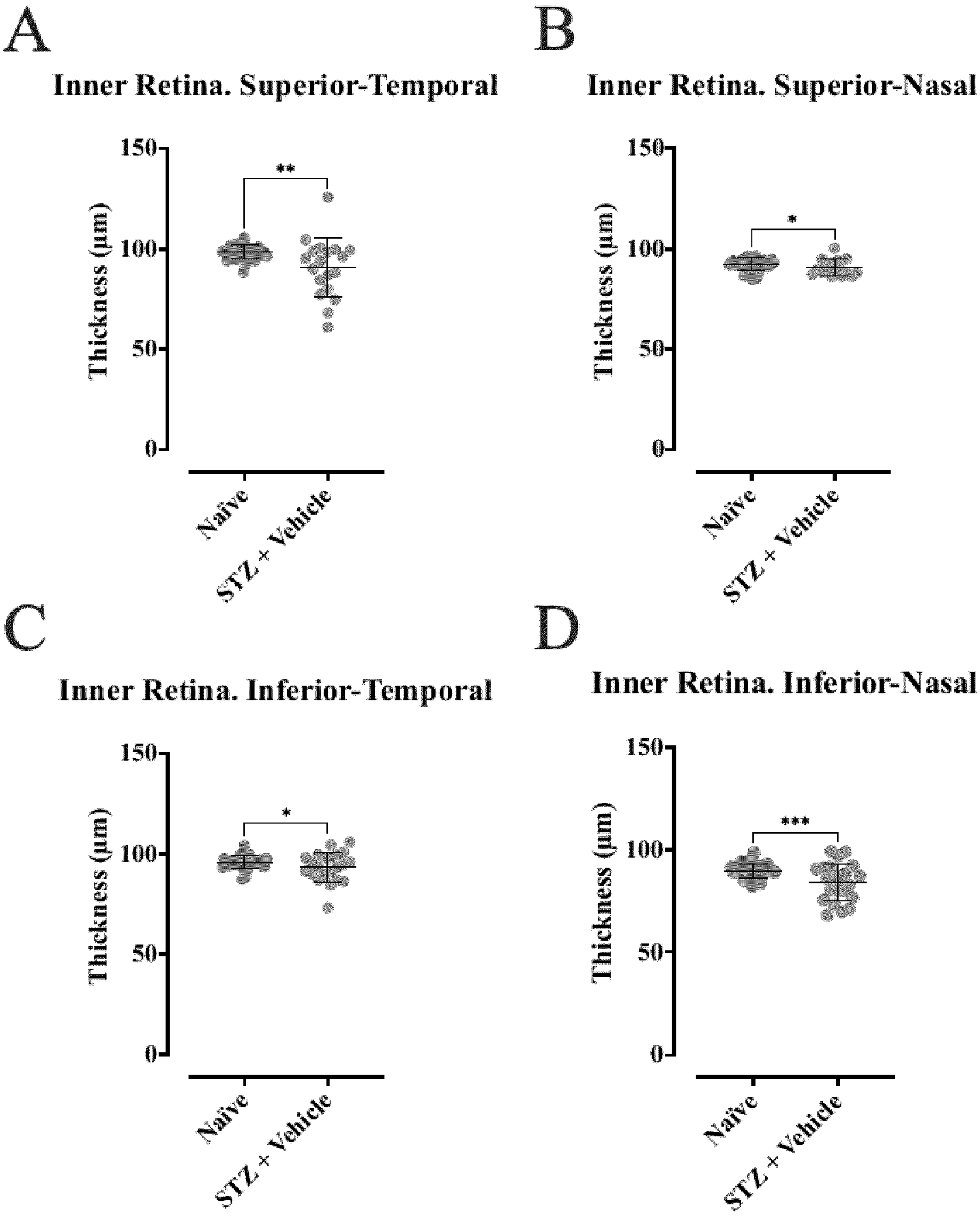
A
Inner Retina. Superior-Temporal
150
100
50
0
Thickness (µm)
**
Naïve
STZ + Vehicle
B
Inner Retina. Superior-Nasal
150
100
50
0
Thickness (µm)
*
Naïve
STZ + Vehicle
C
Inner Retina. Inferior-Temporal
150
100
50
0
Thickness (µm)
*
Naïve
STZ + Vehicle
D
Inner Retina. Inferior-Nasal
150
100
50
0
Thickness (µm)
***
Naïve
STZ + Vehicle

*Figure 8 (continued)*

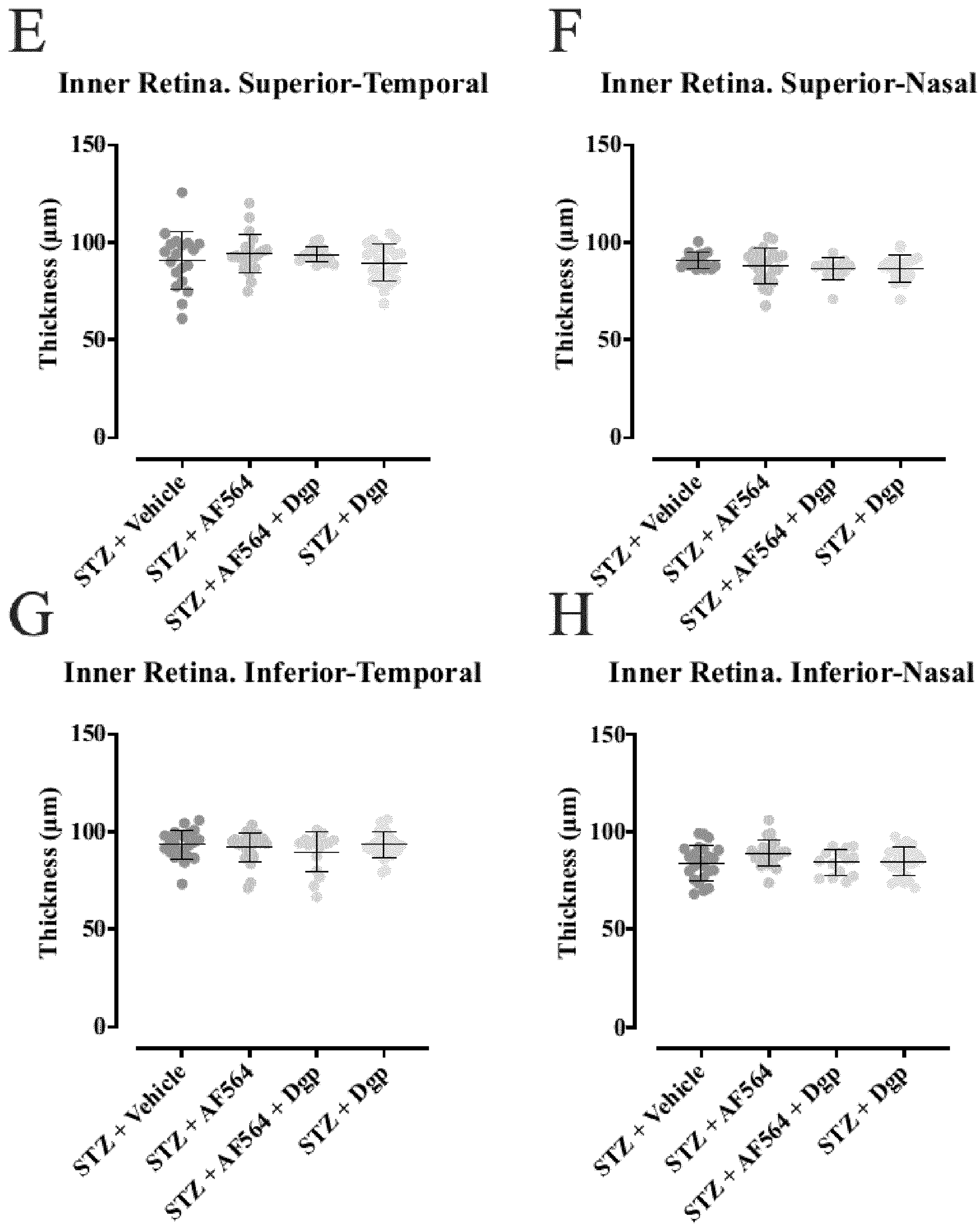

***Figure 9***

A

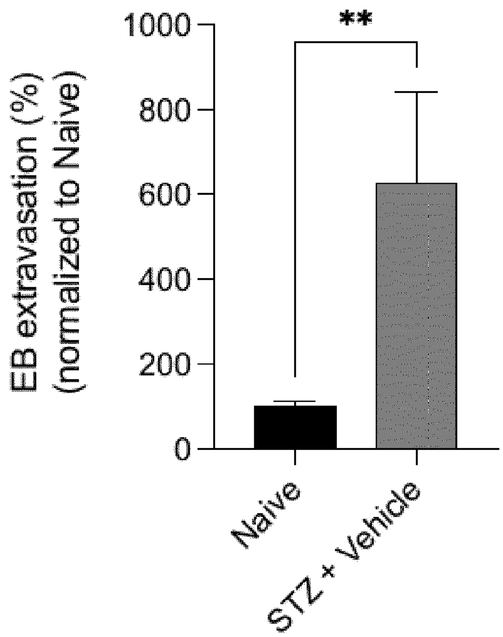
**
1000
800
600
400
200
0
EB extravasation (%)
(normalized to Naive)
Naive
STZ + Vehicle

B

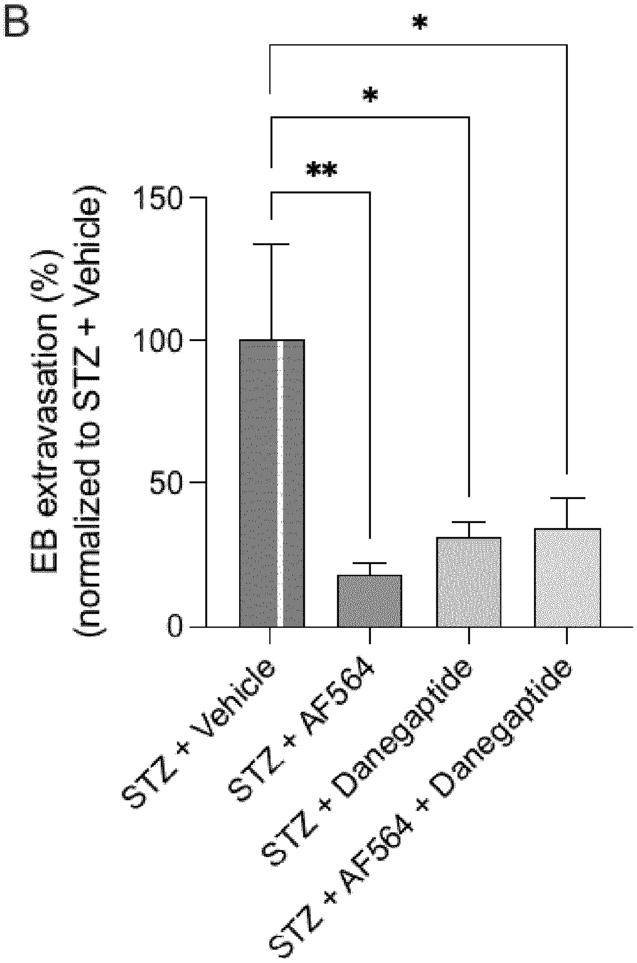
*
*
**
150
100
50
0
EB extravasation (%)
(normalized to STZ + Vehicle)
STZ + Vehicle
STZ + AF564
STZ + Danegaptide
STZ + AF564 + Danegaptide

## *Figure 10*

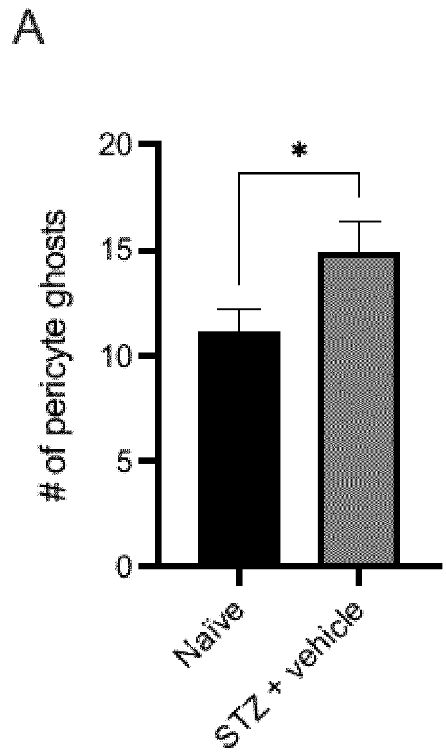
A
20
15
10
5
0
*
# of pericyte ghosts
Naive
STZ + vehicle

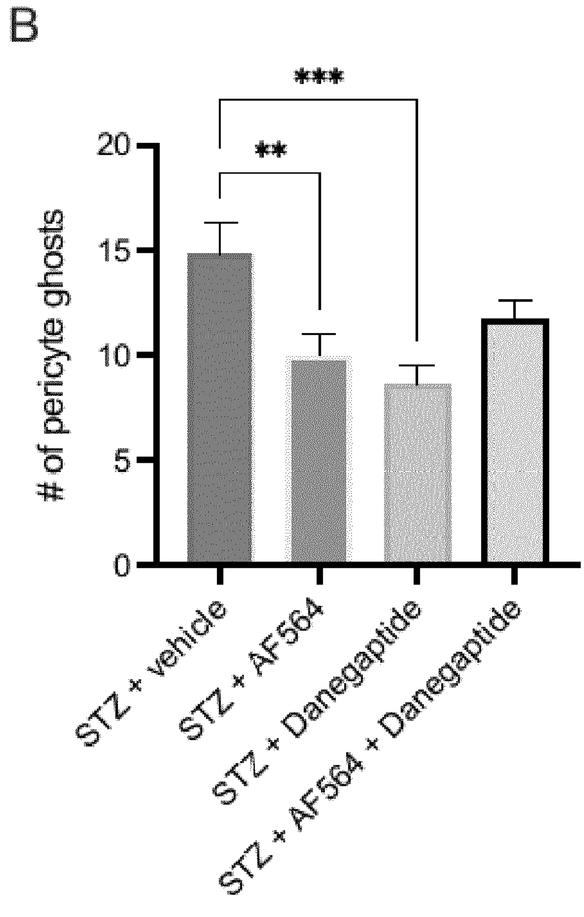
B
20
15
10
5
0
***
**
# of pericyte ghosts
STZ + vehicle
STZ + AF564
STZ + Danegaptide
STZ + AF564 + Danegaptide

# Figure 11

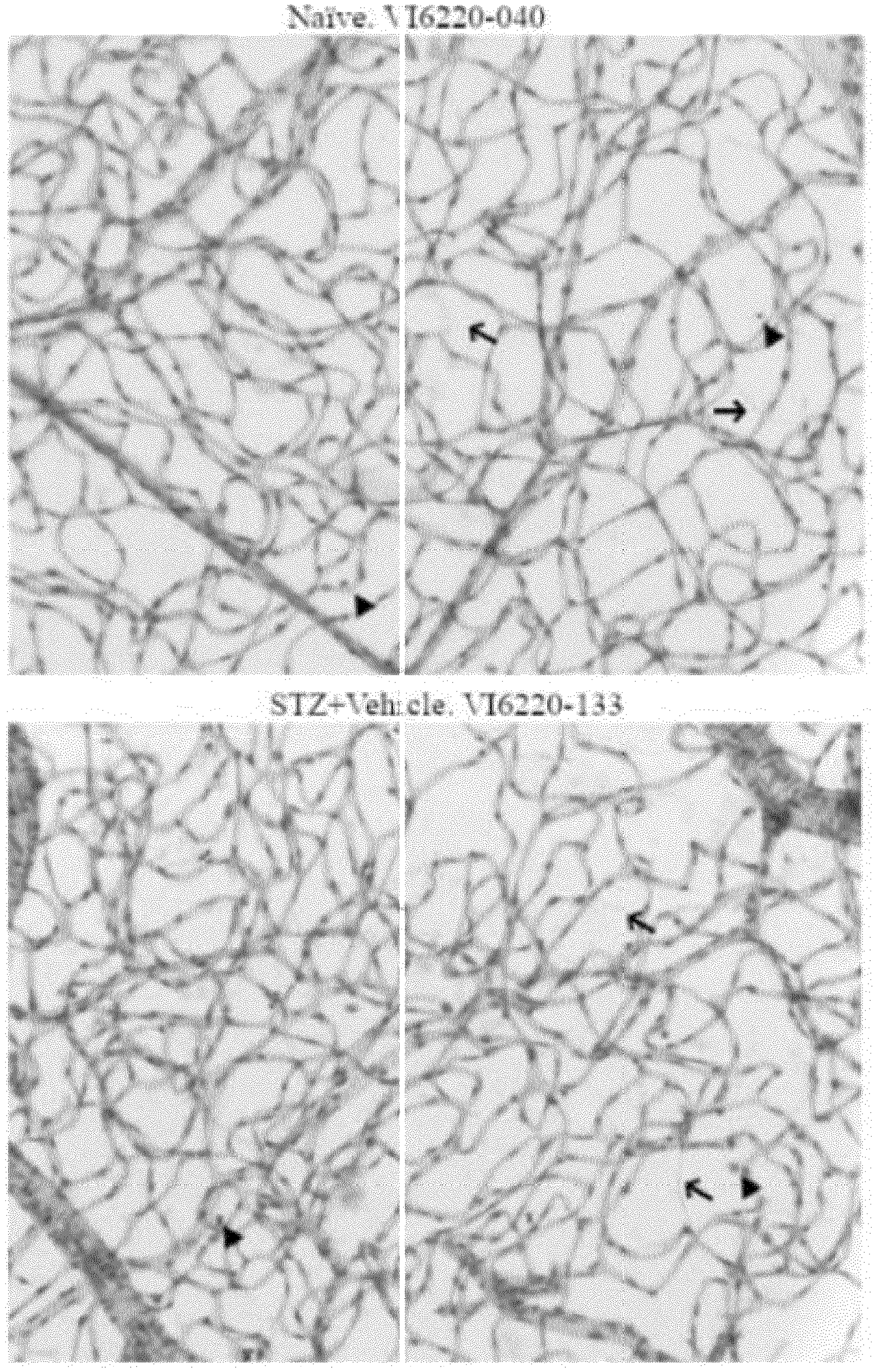

## Figure 11 (continued)

STZ+AF564, VI6220-128

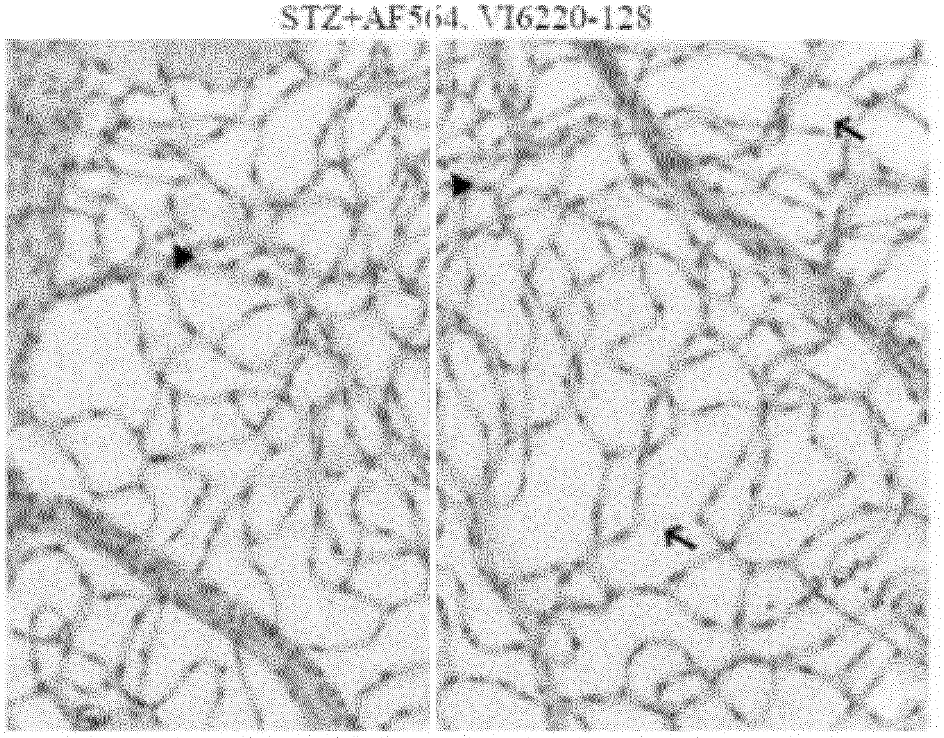

STZ+Dgp, VI6220-93

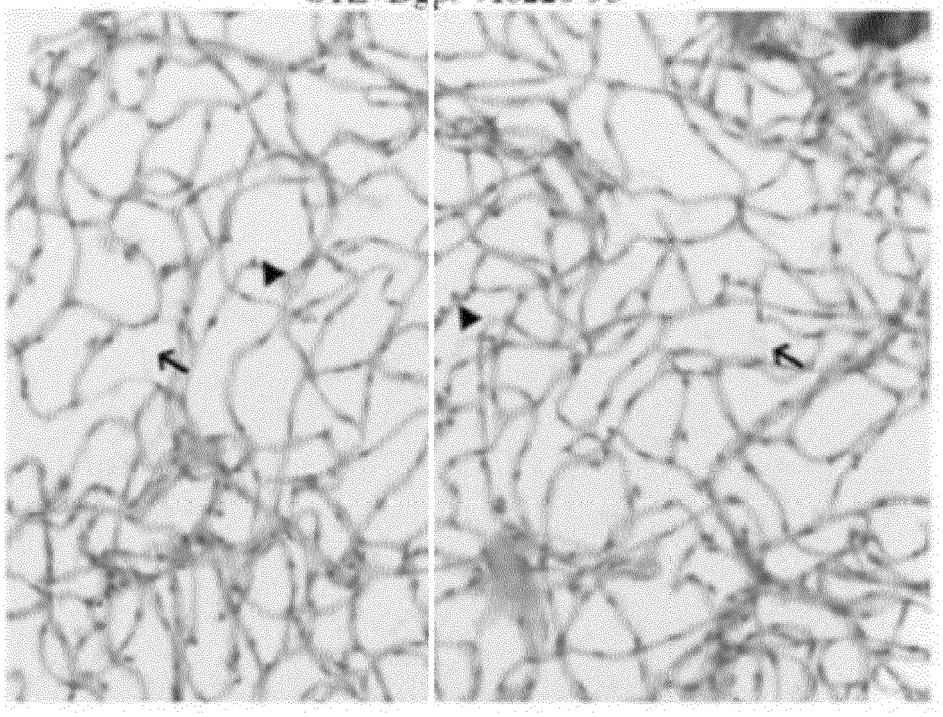

## *Figure 11 (continued)*

STZ+AF564+Dgp. VI6220-127

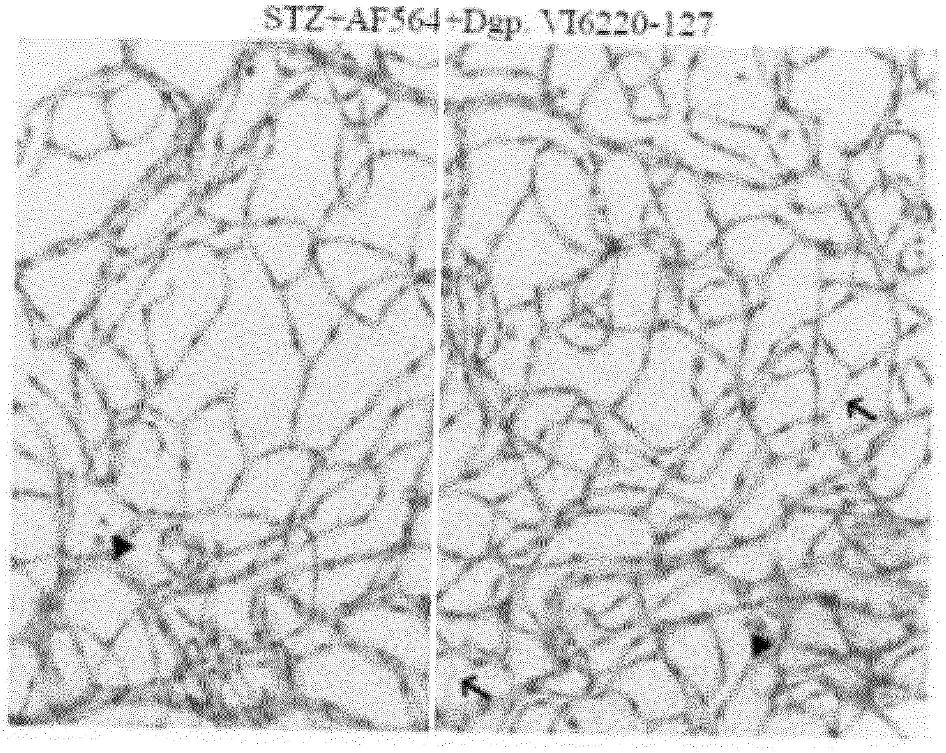

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4775743 A
- WO 02077017 A
- WO 2007078990 A
- WO 2018202865 A
- WO 2008079266 A


### Non-patent literature cited in the description


- Pro-drugs as Novel Delivery Systems. **T. HIGUCHI** ; **V. STELLA**. A.C.S. Symposium Series, and in Bioreversible Carriers in Drug Design. American Pharmaceutical Association and Pergamon Press, 1987, vol. 14
- Remington's Pharmaceutical Sciences. Mark Publishing Company, 1985
- Principles and practice of solid-phase peptide synthesis. **FIELDS et al.** Synthetic Peptides. 2002
- **AONUMA et al.** *Chem. Pharm. Bull. (Tokyo)*, 1980, vol. 28, 3332-3339
- **BUTERA et al.** *J. Med. Chem.*, 2009, vol. 52 (4), 908-911
- **CLAPP et al.** *Acta Diabetologica*, 2019, vol. 56, 1031-1036
- **COUTINHO et al.** *Drug Deliv. Transl. Res.*, 2020, vol. 10 (3), 751-765
- **DHEIN et al.** *Naunyn Schmiedebergs Arch Pharmacol.*, 1994, vol. 350, 174-184
- **FERRIS et al.** *Ophthalmology*, 2013, vol. 120 (4), 844-851
- **FILEK et al.** *Clin. Ophthalmol.*, 2019, vol. 13, 1087-96
- **GONZÁLEZ-CASANOVA et al.** *Int. J. Mol. Sci.*, 2021, vol. 22 (6), 3194
- **HADZIAHMETOVIC et al.** *Front. Cell Dev. Biol.*, 2021, vol. 8, 612812
- **HUDSON et al.** *Neural Regen. Res.*, 2020, vol. 15 (9), 1656-1657
- **KING et al.** *, Int. J. Mol. Sci.*, 2021, vol. 22, 10186
- **MUGISHO et al.** *Biochimica et Biophysica Acta (BBA) - General Subjects*, 2019, vol. 1862 (3), 385-393
- **MULLER et al.** *Eur. J. Pharmacol.*, 1997, vol. 327, 65-72
- **NAYLOR et al.** *, Int. J. Mol. Sci.*, 2020, vol. 21 (1), 211
- **OBERT et al.** *J. Mol. Med. (Berl)*, 2017, vol. 95 (5), 535-552
- **SONG et al.** *Neuro-Oncology*, 2005, vol. 7 (4), 453-464
- **SQUIRES et al.** *, Int. J. Mol. Sci.*, 2021, vol. 22 (6), 2809
- **TERRYN et al.** *Cytometry A*, 2013, vol. 83, 235-241
- **ZINIAUSKAITÉ et al.** *, Ocul Surf.*, 2019, vol. 17, 257-264